(19) Europäisches Patentamt
European Patent Office
Office européen des brevets

(11) **EP 3 126 819 B1**

(12) **EUROPÄISCHE PATENTSCHRIFT**

(45) Veröffentlichungstag und Bekanntmachung des Hinweises auf die Patenterteilung:
**12.12.2018 Patentblatt 2018/50**

(21) Anmeldenummer: **15717119.0**

(22) Anmeldetag: **07.04.2015**

(51) Int Cl.:
*G01N 21/65* (2006.01)      *G01N 33/574* (2006.01)

(86) Internationale Anmeldenummer:
**PCT/EP2015/057502**

(87) Internationale Veröffentlichungsnummer:
**WO 2015/150573 (08.10.2015 Gazette 2015/40)**

(54) **VORRICHTUNG UND VERFAHREN ZUR UNTERSUCHUNG EINER PROBE ZUR PROSTATATUMORERKENNUNG**

DEVICE AND METHOD FOR ANALYSING A SAMPLE FOR THE IDENTIFICATION OF PROSTATE TUMOURS

DISPOSITIF ET PROCÉDÉ SERVANT À ANALYSER UN ÉCHANTILLON AFIN DE DIAGNOSTIQUER UNE TUMEUR DE LA PROSTATE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.04.2014 DE 102014206576**

(43) Veröffentlichungstag der Anmeldung:
**08.02.2017 Patentblatt 2017/06**

(73) Patentinhaber: **CellTool GmbH**
**82347 Bernried (DE)**

(72) Erfinder:
 • SCHUETZE, Karin
  82327 Tutzing (DE)
 • LERNHARDT, Waldemar
  74182 Obersulm-Suelzbach (DE)
 • SCHUETZE, Raimund
  82327 Tutzing (DE)

(74) Vertreter: **Banzer, Hans-Jörg**
**Kraus & Weisert**
**Patentanwälte PartGmbB**
**Thomas-Wimmer-Ring 15**
**80539 München (DE)**

(56) Entgegenhaltungen:
**WO-A1-2010/131045    WO-A1-2011/154143**

 • RACHEL E. KAST ET AL: "Emerging technology: applications of Raman spectroscopy for prostate cancer", CANCER AND METASTASIS REVIEWS, Bd. 33, Nr. 2-3, 9. Februar 2014 (2014-02-09), Seiten 673-693, XP055197344, ISSN: 0167-7659, DOI: 10.1007/s10555-013-9489-6
 • MATTHEW TOLLEFSON ET AL: "Raman spectral imaging of prostate cancer: can Raman molecular imaging be used to augment standard histopathology?", BJU INTERNATIONAL, Bd. 106, Nr. 4, 1. August 2010 (2010-08-01), Seiten 484-488, XP055197347, ISSN: 1464-4096, DOI: 10.1111/j.1464-410X.2010.09185.x
 • P CROW ET AL: "The use of Raman spectroscopy to identify and grade prostatic adenocarcinoma in vitro", BRITISH JOURNAL OF CANCER, Bd. 89, Nr. 1, 7. Juli 2003 (2003-07-07), Seiten 106-108, XP055197369, ISSN: 0007-0920, DOI: 10.1038/sj.bjc.6601059
 • Celltool: "BioRam", , 1. November 2011 (2011-11-01), XP055197356, Gefunden im Internet: URL:http://celltool.de/files/celltool-bioram-flyer_en.pdf [gefunden am 2015-06-22]
 • Bas W. D. De Jong ET AL: "Discrimination between Nontumor Bladder Tissue and Tumor by Raman Spectroscopy", Analytical Chemistry, vol. 78, no. 22, 1 November 2006 (2006-11-01), pages 7761-7769, XP055420927, US ISSN: 0003-2700, DOI: 10.1021/ac061417b

• K. Kong ET AL: "Diagnosis of tumors during tissue-conserving surgery with integrated autofluorescence and Raman scattering microscopy", Proceedings of the National Academy of Sciences, vol. 110, no. 38, 3 September 2013 (2013-09-03), pages 15189-15194, XP055174821, ISSN: 0027-8424, DOI: 10.1073/pnas.1311289110

**Beschreibung**

GEBIET DER ERFINDUNG

Die Erfindung betrifft Vorrichtungen und Verfahren zur Erkennung von Prostatatumoren. Die Erfindung betrifft insbesondere Vorrichtungen und Verfahren, mit denen eine Gewebeprobe ausgewertet wird, um zu erkennen, ob ein aggressiver oder ein nicht aggressiver Prostatatumor vorliegt.

HINTERGRUND

Das Prostatakarzinom gehört zu den häufigsten Tumorerkrankungen des Mannes und ist eine derjenigen Tumore, an dem die meisten männlichen Krebspatienten sterben. Die rechtzeitige Erkennung von Prostatatumoren mit einer Bestimmung, ob es sich um einen bösartigen oder gutartigen Tumor handelt, ist von erheblicher Bedeutung. Verschiedene Techniken sind dazu bekannt. Verdachtsdiagnosen können durch digital-rektale Untersuchungen als Tastbefund erstellt werden. Für eine quantitative Untersuchung können Ultraschalluntersuchung, Magnetresonanztomographie oder Positronen-Emissions-Tomographie (PET) eingesetzt werden. Darüber hinaus können Biomarker, beispielsweise Protein-Biomarker, die Erkennung und Unterscheidung bösartiger Prostatatumore und gutartiger Prostatatumore unterstützen. Einige dieser Biomarker können beispielsweise im Blut, Urin oder Ejakulat nachgewiesen werden. Bei der Untersuchung einer bioptisch entnommenen Gewebeprobe wird anhand der Gewebeprobe bestimmt, ob Prostatakrebs vorliegt. Die Erkennung von Prostatatumoren, und die Bestimmung ob ein aggressiver Tumor oder nicht aggressiver Tumor vorliegt, ist insbesondere für eine Entscheidung von Bedeutung, ob eine weitere Beobachtung (so genannte "active surveillance") angezeigt ist oder Therapien wie Prostatektomie, eine Strahlentherapie, eine Hormontherapie oder eine Chemotherapie durchgeführt werden müssen.

Die effiziente und objektive Untersuchung von Proben, beispielsweise Blut, Urin, Ejakulat, Biopsaten oder Gewebeschnitten, bleibt jedoch eine Herausforderung. Viele der herkömmlichen Ansätze zur Untersuchung der Proben sind aufwändig, kostspielig und oft nicht hinreichend objektiv.

Insbesondere besteht bei der Untersuchung von Biopsaten häufig das Problem, dass die Biopsate gerade bei kleinen Prostatatumoren ausschließlich Gewebe enthalten können, das weit von dem Prostatatumor entfernt liegt. In diesen Fällen erlauben herkömmliche Untersuchungsmethoden häufig keine ausreichend zuverlässige Erkenntnis, ob ein aggressiver oder nicht-aggressiver Prostatatumor vorliegt. Kast et al., 2014, offenbaren die Verwendung von Raman-Spektroskopie im klinischen Umfeld.

ZUSAMMENFASSUNG

Es besteht ein Bedarf an Vorrichtungen und Verfahren zum Untersuchen einer Probe, mit denen Prostatatumore erkannt werden können. Es besteht insbesondere ein Bedarf an derartigen Vorrichtungen und Verfahren, bei denen anhand quantitativer Messwerte in objektiver Weise bestimmt werden kann, ob ein aggressiver oder ein nicht aggressiver Prostatatumor vorliegt.

Nach Ausführungsbeispielen der Erfindung wird zur Untersuchung einer Probe eine Raman-Spektroskopie ausgeführt. Ein oder mehrere Raman-Spektren können analysiert werden, um anhand des Raman-Spektrums oder anhand einer ortsabhängigen Veränderung von Raman-Spektren einen durch Raman-Spektroskopie erfassten Fingerabdruck eines aggressiven oder nicht aggressiven Prostatatumors zu erhalten. Beispielsweise können ein oder mehrere Raman-Spektren analysiert werden, um anhand der Anwesenheit oder Abwesenheit von bestimmten Biomolekülen Prostatatumore und optional deren Aggressivität zu erkennen und/oder deren räumliche Verteilung zu ermitteln. Die Probe kann eine Gewebeprobe, beispielsweise ein Biopsat oder ein Gewebeschnitt, sein.

Durch die Auswertung eines oder mehrerer Raman-Spektren kann die Probe objektiv und quantitativ untersucht werden. Es kann ein Vergleich mit in einer Datenbank hinterlegten Referenzspektren vorgenommen werden, um zu bestimmen, ob ein bösartiger oder gutartiger Prostatatumor vorliegt. Zusätzliche Aussagen können aus den Raman-Spektren ermittelt werden, beispielsweise über die Aggressivität oder das Stadium des Prostatatumors (so genanntes "Staging" oder "Scoring").

Die Referenzspektren können ermittelt sein aus Gewebeproben von Prostataektomie, von Biopsaten der Prostata, von Prostata-Krebszellen aus Zellkulturen oder durch Messen der Raman-Spektren von Biomarkern. Die Referenzspektren können Raman-Spektren oder Charakteristika von Raman-Spektren des Stroma der Prostata umfassen. Die Referenzspektren können durch einen Vergleich von Raman-Spektren von normalem Prostatagewebe mit krebsbefallenem Prostatagewebe erstellt sein. Entsprechende Referenzspektren können für Stroma und/oder für Gewebe, das nicht Stroma der Prostata ist, erstellt werden.

Nach einem Ausführungsbeispiel wird eine Vorrichtung zum Erkennen eines Prostatatumors angegeben. Die Vorrichtung umfasst ein Raman-Spektroskopiesystem zum Erfassen wenigstens eines Raman-Spektrums einer Probe.

Die Vorrichtung umfasst eine elektronische Auswerteeinrichtung, die eingerichtet ist, um abhängig von einer Auswertung des wenigstens einen Raman-Spektrum den Prostatatumor zu erkennen.

Die elektronische Auswerteeinrichtung kann eingerichtet sein, um durch die Auswertung des wenigstens einen Raman-Spektrums zu erkennen, ob ein aggressiver Prostatatumor oder ein nicht aggressiver Prostatatumor vorliegt. Die elektronische Auswerteeinrichtung kann eingerichtet sein, um eine Intensität des Raman-Signals bei wenigstens einer Wellenzahl auszuwerten, die einem Biomarker für einen aggressiven Prostatatumor oder für einen nicht aggressiven Prostatatumor zugeordnet ist.

Die elektronische Auswerteeinrichtung kann eingerichtet sein, um durch die Auswertung des wenigstens einen Raman-Spektrums zu erkennen, ob der Prostatatumor bösartig ist. Die elektronische Auswerteeinrichtung kann eingerichtet sein, um durch die Auswertung des wenigstens einen Raman-Spektrums auch dann zu erkennen, ob der Prostatatumor bösartig ist, wenn ein Gleason-Score von zum Beispiel 6 keine eindeutige Zuordnung zu bösartig oder nicht bösartig ermöglicht.

Die elektronische Auswerteeinrichtung kann eingerichtet sein, um durch die Auswertung des wenigstens einen Raman-Spektrums eine Aggressivität des Prostatatumors zu erkennen. Die elektronische Auswerteeinrichtung kann eingerichtet sein, um durch die Auswertung des wenigstens einen Raman-Spektrums eine Beurteilung eines bösartigen Prostatatumors (so genanntes "Grading" des Prostatatumors) vorzunehmen.

Die elektronische Auswerteeinrichtung kann eingerichtet sein, um durch die Auswertung des wenigstens einen Raman-Spektrums zu ermitteln, in welchem Stadium sich der Prostatatumor befindet (so genanntes "Staging" des Prostatatumors).

Die Vorrichtung kann eingerichtet sein, um an einer Mehrzahl von Positionen einer Gewebeprobe jeweils mindestens ein Raman-Spektrum zu erfassen. Die Vorrichtung kann wenigstens einen steuerbaren Motor umfassen, um eine Relativbewegung zwischen der Probe und optischen Komponenten des Raman-Spektroskopiesystems hervorzurufen, um automatisch Raman-Spektren an einer Mehrzahl von Positionen der Gewebeprobe zu erfassen. Die Mehrzahl von Positionen und der Abstand kann auch benutzerdefiniert festlegbar sein. Die Mehrzahl von Positionen und der Abstand kann durch das System automatisch ermittelt werden, beispielsweise durch eine entsprechende Konfigurationen vorgegeben sein, die in Hardware, Software oder Firmware implementiert sein kann.

Die Vorrichtung kann eingerichtet sein, um die Mehrzahl von Positionen so festzulegen, dass für einzelne Zellen in einer Gewebeprobe jeweils ein Raman-Spektrum erfasst werden kann. Alternativ oder zusätzlich kann die Vorrichtung eingerichtet sein, um eine Mehrzahl von Positionen so festzulegen, dass für einzelne subzelluläre Bereiche, beispielsweise für Zellkerne und Cytoplasma, in einer Gewebeprobe jeweils ein Raman-Spektrum erfasst wird.

Die Vorrichtung kann wenigstens einen steuerbaren Motor umfassen, um eine Relativbewegung zwischen der Probe und optischen Komponenten des Raman-Spektroskopiesystems hervorzurufen, um automatisch Raman-Spektren an der Mehrzahl von Positionen der Gewebeprobe zu erfassen. Die Mehrzahl von Positionen kann auch benutzerdefiniert festlegbar sein.

Die elektronische Auswerteeinrichtung kann eingerichtet sein, um die an der Mehrzahl von Positionen erfassten Raman-Spektren auszuwerten. Dadurch können insbesondere Veränderungen von Raman-Peaks automatisch erkannt werden, die mit gutartigen oder bösartigen Prostatatumoren in Verbindung stehen.

Die elektronische Auswerteeinrichtung kann eingerichtet sein, um für die an der Mehrzahl von Positionen erfassten Raman-Spektren jeweils eine statistische Analyse, beispielsweise eine Hauptkomponentenanalyse ("Principal Component Analysis", PCA) oder eine hierarchische oder nicht hierarchische Clusteranalyse ausführen.

Anhand des Ergebnisses der statistischen Analyse kann die elektronische Auswerteeinrichtung eine Bewertung vornehmen, ob ein aggressiver oder ein nicht aggressiver Prostatatumor vorhanden ist. Die Hauptkomponentenanalyse oder Clusteranalyse kann eine automatische Unterscheidung von aggressiven oder ein nicht aggressiven Prostatatumor anhand der Raman-Spektren ermöglichen.

Die elektronische Auswerteeinrichtung kann eingerichtet sein, um eine ortsabhängige Veränderung eines Raman-Signals aus den an der Mehrzahl von Positionen erfassten Raman-Spektren zu ermitteln, um zu bestimmen, ob ein aggressiver oder ein nicht aggressiver Prostatatumor vorliegt. Es kann die ortsabhängige Veränderung des Raman-Signals mindestens eines Biomarkers für aggressive Prostatatumore erfasst werden, um zu ermitteln, ob ein aggressiver Prostatatumor oder ein nicht aggressiver Prostatatumor vorliegt. Es kann die ortsabhängige Veränderung des Resultats der statistischen Analyse der an mehreren Positionen erfassten Raman-Spektren bestimmt werden, um zu ermitteln, ob ein aggressiver Prostatatumor oder ein nicht aggressiver Prostatatumor vorliegt. Auf diese Weise kann aus der Gesamtheit des Raman-Spektrums ermittelt werden, ob ein aggressiver oder ein nicht aggressiver Prostatatumor vorliegt. Eine Zuordnung von Raman-Peaks zu einzelnen Biomarkern ist möglich, aber nicht unbedingt erforderlich. Eine Unterscheidung aggressiver und nicht aggressiver Prostatatumore ist in einfacher Weise möglich. Insbesondere kann durch eine Analyse eines Raman-Spektrums oder mehrerer Raman-Spektren, das bzw. die an Gewebe oder daraus gewonnenen Substanzen erfasst wird bzw. werden, ermittelt werden, ob ein aggressiver oder ein nicht aggressiver Prostatatumor vorliegt.

Eine Unterscheidung aggressiver oder nicht aggressiver Prostatatumore kann erfolgen, ohne dass es unbedingt erforderlich ist, einzelne Raman-Peaks Biomarkern oder anderen Stoffen zuzuordnen. Vielmehr kann aus der Gesamtheit des Raman-Spektrums ermittelt werden, ob ein aggressiver oder nicht aggressiver Prostatatumor vorliegt. Mit einer statistischen Analyse, z.B. einer PCA oder Clustertechniken, kann ein Muster oder können mehrere Muster in dem Spektrum als Unterscheidungskriterium zur Unterscheidung aggressiver und nicht aggressiver Prostatatumore verwendet werden. Derartige Muster können durch die Lage mehrerer Raman-Peaks, deren Höhe, und/oder die Steilheit der Flankendefiniert werden.

Alternativ oder zusätzlich kann auch die Reaktion von Prostatazellen, Stromazellen oder anderen Bestandteilen der Probe auf von einem Prostatatumor erzeugte Stoffe erkannt werden.

Die elektronische Auswerteeinrichtung kann eingerichtet sein, um einen Gradienten der örtlichen Veränderung ortsaufgelöst zu bestimmen. Der Gradient kann der Gradient einer Intensität, d.h. der Amplitude, eines Raman-Peaks. Der Gradient kann der Gradient einer Höhe, Flankensteilheit von Raman-Peaks und/oder Kombination der Peaks. Der Gradient kann der Gradient einer Lage des Ergebnisses einer Hauptkomponentenanalyse oder eine hierarchischen oder nicht hierarchischen Cluster-Analyse in einem mehrdimensionalen Datenraum sein. Daraus kann eine Beurteilung des Prostatatumors durch die elektronische Auswerteeinrichtung vorgenommen werden. Alternativ oder zusätzlich können Rückschlüsse auf die Position des bösartigen Prostatatumors gezogen werden.

Die elektronische Auswerteeinrichtung kann eingerichtet sein, um den Gradienten ortsaufgelöst zu ermitteln.

Die elektronische Auswerteeinrichtung kann eingerichtet sein, um eine Position des Prostatatumors in der Gewebeprobe oder relativ zu der Gewebeprobe durch die Auswertung der an der Mehrzahl von Positionen erfassten Raman-Spektren zu ermitteln. Die elektronische Auswerteeinrichtung kann eingerichtet sein, um die Position eines bösartigen Prostatatumors anhand der örtlichen Veränderung von Raman-Signalen auch dann zu bestimmen, wenn keine Krebszellen in der Gewebeprobe enthalten sind. Die elektronische Auswerteeinrichtung kann eingerichtet sein, um die Position eines aggressiven Prostatatumors anhand der örtlichen Veränderung von Raman-Signalen auch dann zu bestimmen, wenn die Gewebeprobe eine Gewebeprobe des Stroma der Prostata ist.

Die elektronische Auswerteeinrichtung kann eingerichtet sein, um die an der Mehrzahl von Positionen erfassten Raman-Spektren mit gespeicherten Referenz-Spektren zu vergleichen. Die Referenzspektren können ortsaufgelöst ermittelte Raman-Spektren von Proben mit bösartigen Prostatatumoren, von Proben mit nichtaggressiven Prostatatumoren und/oder von Proben ohne Prostatatumore enthalten.

Die elektronische Auswerteeinrichtung kann eingerichtet sein, um die an der Mehrzahl von Positionen erfassten Raman-Spektren einer statistischen Analyse, beispielsweise einer Hauptkomponentenanalyse oder einer hierarchischen oder nicht hierarchischen Cluster-Analyse, zu unterziehen und das Ergebnis mit einer statischen Analyse der Referenzspektren zu vergleichen.

Die Gewebeprobe kann ein histologischer Schnitt eines Biopsats sein. Das Biopsat kann ein mit Stanzbiopsie gewonnenes Biopsat sein.

Die Gewebeprobe kann ein histologischer Schnitt von einem Teil oder mehreren Teilen einer chirurgisch entfernten Prostata sein.

Die Vorrichtung kann eingerichtet sein, um für Gewebeproben mehrerer Biopsate jeweils ortsaufgelöst mehrere Raman-Spektren zu erfassen. Die für die mehreren Biopsate ausgewerteten Raman-Spektren können verwendet werden, um zu bestimmen, ob ein gutartiger Tumor oder ein bösartiger Tumor vorliegt. Die für die mehreren Biopsate ausgewerteten Raman-Spektren können verwendet werden, um zu bestimmen, ob ein aggressiver Tumor oder ein nicht aggressiver Tumor vorliegt.

Das Biopsat kann ein Biopsat des Stroma der Prostata sein.

Die Probe ist vorzugsweise eine Gewebeprobe. Die Vorrichtung kann eingerichtet sein, um durch Raman-Spektroskopie zu ermitteln, ob Biomoleküle bzw. Biomarker vorhanden sind, die das Vorliegen einen aggressiven Prostatatumors anzeigen.

Die Biomoleküle, die mit der Raman-Spektroskopie identifiziert werden, können beispielsweise DNA oder Proteine, also genetische oder molekulare Biomarker oder eine Mischung daraus sein. Die Biomarker können Protein-Biomarker sein. Die daraus ermittelten charakteristischen Raman-Spektren können als ein "photonischer Fingerabdruck" eingesetzt werden, der einen photonischen Biomarker für die Erkennung aggressiver Prostatatumore bereitstellt. Diese können als Referenz für weitere Untersuchungen dienen. Eine Zuordnung derartiger photonischer Fingerabdrücke zu einzelnen Biomarkern ist möglich, aber nicht unbedingt erforderlich, um beispielsweise aggressive von nicht aggressiven Tumoren zu unterscheiden. Beispielsweise kann eine statistische Analyse, wie eine Hauptkomponentenanalyse oder eine Cluster-Analyse auf ein oder mehrere erfasste Raman-Spektren angewandt werden, um zu erkennen, ob das erfasste Raman-Spektrum einem aggressiven oder einem nicht aggressiven Tumor zuzuordnen ist.

Die elektronische Auswerteeinrichtung kann eingerichtet sein, um durch die Auswertung des wenigstens einen Raman-Spektrums eine Aggressivität des Prostatatumors zu bestimmen.

Die elektronische Auswerteeinrichtung kann eingerichtet sein, um anhand des erfassten Raman-Spektrums bei einer oder mehreren Wellenzahlen oder Wellenzahlbereichen die Aggressivität des Prostatatumors zu bestimmen. Die ein oder mehreren Wellenzahlen oder Wellenzahlenbereiche können ausgewählt sein aus einzelnen oder mehreren Wellenzahlgruppen.

Ein Verfahren zum Auswerten einer Probe zum Erkennen eines Prostatatumors umfasst ein Erfassen wenigstens eines Raman-Spektrums der Probe und ein Erkennen des Prostatatumors durch Auswerten des wenigstens einen Raman-Spektrums.

Die Probe ist eine Gewebeprobe, beispielsweise ein Gewebeschnitt eines durch Stanzbiopsie gewonnenes Biopsat sein.

Die Gewebeprobe kann ein histologischer Schnitt von einem Teil oder mehreren Teilen einer chirurgisch entfernten Prostata sein.

Raman-Spektren können an einer Mehrzahl von Positionen der Gewebeprobe erfasst werden. Die an der Mehrzahl von Positionen erfassten Raman-Spektren können zum Erkennen des Prostatatumors ausgewertet werden.

Für die an der Mehrzahl von Positionen erfassten Raman-Spektren kann jeweils eine statistische Analyse, beispielsweise eine Hauptkomponentenanalyse oder eine hierarchische oder nicht hierarchische Clusteranalyse ausgeführt werden.

Die Raman-Spektren können an einer Mehrzahl von subzellulären Positionen der Zellen in einer Gewebeprobe erfasst werden. Die an der Mehrzahl von subzellulären Positionen erfassten Raman-Spektren können zum Erkennen des Prostatatumors ausgewertet werden.

Abhängig von den an der Mehrzahl von Positionen erfassten Raman-Spektren kann eine ortsaufgelöste Veränderung von Raman-Signalen bestimmt werden, die aggressiven Prostatatumoren zugeordnet sind. Es kann die ortsabhängige Veränderung des Resultats der statistischen Analyse der an mehreren Positionen erfassten Raman-Spektren bestimmt werden, um zu ermitteln, ob ein aggressiver Prostatatumor oder ein nicht aggressiver Prostatatumor vorliegt. Auf diese Weise kann aus der Gesamtheit des Raman-Spektrums ermittelt werden, ob ein aggressiver oder ein nicht aggressiver Prostatatumor vorliegt. Eine Zuordnung von Raman-Peaks zu einzelnen Biomarkern ist möglich, aber nicht unbedingt erforderlich. Eine Unterscheidung aggressiver und nicht aggressiver Prostatatumore ist in einfacher Weise möglich.

Abhängig von den an der Mehrzahl von Positionen erfassten Raman-Spektren kann bestimmt werden, wie sich ein Biomarker ortsabhängig verändert.

Mit dem Verfahren kann eine Position des Prostatatumors in der Gewebeprobe oder relativ zu der Gewebeprobe bestimmt werden.

Mit dem Verfahren kann eine Aggressivität des Prostatatumors bestimmt werden. Zur Bestimmung der Aggressivität kann das Ergebnis einer statistischen Analyse des Raman-Spektrums mit den Ergebnissen dieser statistischen Analyse für Referenzspektren, die aggressiven und nicht aggressiven Prostatatumoren zugeordnet sind, verglichen werden.

Mit dem Verfahren kann ein bzw. können mehrere "photonischer Fingerabdrücke" der Probe gewonnen werden, die die erfasste Raman-Intensität an einer Mehrzahl N von Wellenzahlen, beispielsweise N > 100 Wellenzahlen, definieren. Eine Zuordnung derartiger photonischer Fingerabdrücke zu einzelnen Biomarkern ist möglich, aber nicht unbedingt erforderlich, um beispielsweise aggressive von nicht aggressiven Tumoren zu unterscheiden. Beispielsweise kann eine statistische Analyse, wie eine Hauptkomponentenanalyse oder eine Cluster-Analyse auf ein oder mehrere erfasste Raman-Spektren angewandt werden, um zu erkennen, ob das erfasste Raman-Spektrum einem aggressiven oder einem nicht aggressiven Tumor zuzuordnen ist.

Das Verfahren kann von der Vorrichtung nach einem Ausführungsbeispiel automatisch ausgeführt werden.

Die Verfahren nach Ausführungsbeispielen können fern vom menschlichen oder tierischen Körper ausgeführt werden. Die Vorrichtung nach Ausführungsbeispielen kann für eine Untersuchung der Probe verwendet werden, wobei die Untersuchung fern vom menschlichen oder tierischen Körper ausgeführt wird.

Die Vorrichtungen und Verfahren nach Ausführungsbeispielen können so ausgestaltet sein, dass die Gewinnung der Probe, insbesondere die Gewinnung eines Biopsats oder Gewebeschnitts, nicht Teil der beanspruchten Vorrichtungen und Verfahren ist.

Vorrichtungen und Verfahren nach Ausführungsbeispielen erlauben eine objektive Erkennung aggressiver Prostatatumore und deren Unterscheidung von nichtaggressiven Prostatatumoren durch quantitative Auswertung wenigstens eines Raman-Spektrums einer Probe.

KURZE BESCHREIBUNG DER FIGUREN

Die Erfindung wird nachfolgend unter Bezugnahme auf die Zeichnung anhand bevorzugter Ausführungsbeispiele weiter erläutert.

Figur 1 zeigt eine schematische Darstellung einer Vorrichtung nach einem Ausführungsbeispiel.

Figur 2 zeigt die ortsaufgelöste Erfassung von Raman-Spektren einer Probe nach einem Ausführungsbeispiel.

Figur 3 zeigt die ortsaufgelöste Erfassung von Raman-Spektren einer Probe nach einem Ausführungsbeispiel.

Figur 4 zeigt ein beispielhaftes Raman-Spektrum, das von einer Vorrichtung nach einem Ausführungsbeispiel ausgewertet wird.

Figur 5 zeigt ein weiteres beispielhaftes Raman-Spektrum, das von einer Vorrichtung nach einem Ausführungsbeispiel ausgewertet wird.

Figur 6 zeigt ein weiteres beispielhaftes Raman-Spektrum, das von einer Vorrichtung nach einem Ausführungsbeispiel ausgewertet wird.

Figur 7 illustriert eine Verarbeitung erfasster Raman-Spektren durch eine Vorrichtung nach einem Ausführungsbeispiel.

Figur 8 illustriert eine durch eine Vorrichtung nach einem Ausführungsbeispiel analysierte Probe zur Veranschaulichung der Funktionsweise der Vorrichtung.

Figur 9 illustriert eine Verarbeitung erfasster Raman-Spektren durch eine Vorrichtung nach einem Ausführungsbeispiel.

Figur 10 illustriert eine Verarbeitung erfasster Raman-Spektren durch eine Vorrichtung nach einem Ausführungsbeispiel.

Figur 11 illustriert eine Verarbeitung erfasster Raman-Spektren durch eine Vorrichtung nach einem Ausführungsbeispiel.

Figur 12 illustriert eine Verarbeitung erfasster Raman-Spektren durch eine Vorrichtung nach einem Ausführungsbeispiel.

Figur 13 illustriert die Lage von durch Stanzbiopsie entnommenen Proben, die von einer Vorrichtung nach einem Ausführungsbeispiel ausgewertet werden.

Figur 14 illustriert eine Verarbeitung erfasster Raman-Spektren durch eine Vorrichtung nach einem Ausführungsbeispiel.

Figur 15 ist ein Flussdiagramm eines Verfahrens nach einem Ausführungsbeispiel.

BESCHREIBUNG VON AUSFÜHRUNGSBEISPIELEN

Ausführungsbeispiele werden unter Bezugnahme auf die Figuren beschrieben, in denen ähnliche Bezugszeichen ähnliche Merkmale bezeichnen. Die Merkmale der verschiedenen beschriebenen Ausführungsformen können miteinander kombiniert werden, sofern dies in der nachfolgenden Beschreibung nicht ausdrücklich ausgeschlossen ist. Vorrichtungen und Verfahren nach Ausführungsbeispielen können zur Untersuchung einer Probe eingesetzt werden um zu bestimmen, ob ein Prostatatumor bösartig ist. Vorrichtungen und Verfahren nach Ausführungsbeispielen können zur automatischen Beurteilung eines bösartigen Prostatatumors eingesetzt werden um die Aggressivität des Prostatatumors zu bestimmen.

Bei Vorrichtungen und Verfahren nach Ausführungsbeispielen wird wenigstens ein Raman-Spektrum einer Probe erfasst. Die Probe kann beispielsweise ein Gewebeschnitt eines durch Stanzbiopsie gewonnenen Biopsats, ein Gewebeschnitt von einer chirurgisch entfernten Prostata sein oder daraus gewonnen sein. Wenigstens ein Raman-Spektrum wird ausgewertet, um zu bestimmen, ob ein bösartiger Prostatatumor vorliegt. Zusätzliche Bestimmungen, beispielsweise zur Beurteilung ("Grading") oder Stadienbestimmung ("Staging") können anhand eines oder mehrerer Raman-Spektren vorgenommen werden.

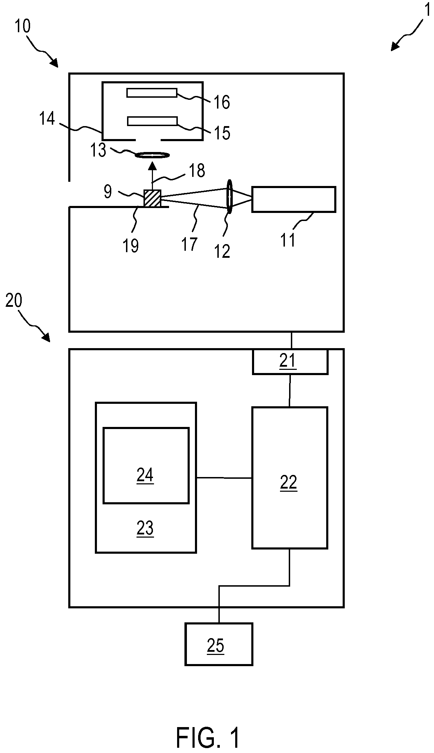

Figur 1 ist eine schematische Darstellung einer Vorrichtung 1 nach einem Ausführungsbeispiel. Die Vorrichtung 1 ist eingerichtet, um zum Erkennen eines Prostatatumors, insbesondere eines bösartigen Prostatatumors, eine Probe 9 mit Raman-Spektroskopie zu untersuchen. Die entsprechende Erkennung erfolgt anhand wenigstens eines Raman-Spektrums, das die Vorrichtung 1 erfasst und automatisch auswerten kann.

Die Vorrichtung 1 umfasst ein Raman-Spektroskopiesystem 10 und eine Auswerteeinrichtung 20. Das Raman-Spektroskopiesystem 10 ist eingerichtet, um ein Raman-Spektrum der Probe 9 zu erfassen. Die Probe 9 kann beispielsweise aus einem Biopsat oder einem Gewebeschnitt gewonnen werden, wobei die Gewinnung der Probe insbesondere durch Stanzbiopsie oder anderer Entnahme von Material nicht Gegenstand der Verfahren nach Ausführungsbeispielen ist.

Die Probe 9 kann Zellen des Stromas der Prostata enthalten. Die Probe 9 kann Zellen der Prostata enthalten.

Das Raman-Spektroskopiesystem 10 umfasst einen Lichtquelle 11, die insbesondere ein Laser sein kann. Die Lichtquelle 11 ist eingerichtet, um einen Anregungsstrahl 17 auszugeben. Ein Ramanspektrometer 14 empfängt an der Probe 9 durch Stokes-Prozesse und/oder Anti-Stokes-Prozesse gestreutes Licht 18. Das Ramanspektrometer 14 kann ein diffraktives Element 15 und einen Bildsensor 16 umfassen, um das Raman-Spektrum der Probe 9 zu erfassen. Das Raman-Spektroskopiesystem 10 kann in an sich bekannter Weise weitere Elemente umfassen, beispielsweise fokussierende optische Elemente 12, 13, die als Linsen ausgestaltet sein können, und/oder Blenden.

Die Vorrichtung 1 umfasst eine Auswerteeinrichtung 20. Die Auswerteeinrichtung 20 kann ein Computer sein oder kann einen Computer umfassen. Die Auswerteeinrichtung 20 ist mit dem Raman-Spektroskopiesystem 10 gekoppelt. Die Auswerteeinrichtung 20 kann die Erfassung des Raman-Spektrums durch das Raman-Spektroskopiesystem 10 steuern. Die Auswerteeinrichtung 20 kann das Raman-Spektroskopiesystem 10 so steuern, dass Raman-Spektren ortsaufgelöst an mehreren Stellen der Probe 9 erfasst werden.

Die Auswerteeinrichtung 20 weist eine Schnittstelle 21 auf, um Daten von dem Bildsensor 16 des Raman-Spektroskopiesystems 10 zu empfangen. Die Auswerteeinrichtung weist eine integrierte Halbleiterschaltung 22 auf, die einen Prozessor oder Controller umfassen kann und die eingerichtet ist, um das erfasste Raman-Spektrum auszuwerten, um zu bestimmen. Die integrierte Halbleiterschaltung 22 ist eingerichtet, um anhand des wenigstens einen Raman-Spektrums zu bestimmen, ob ein bösartiger Prostatatumor vorliegt.

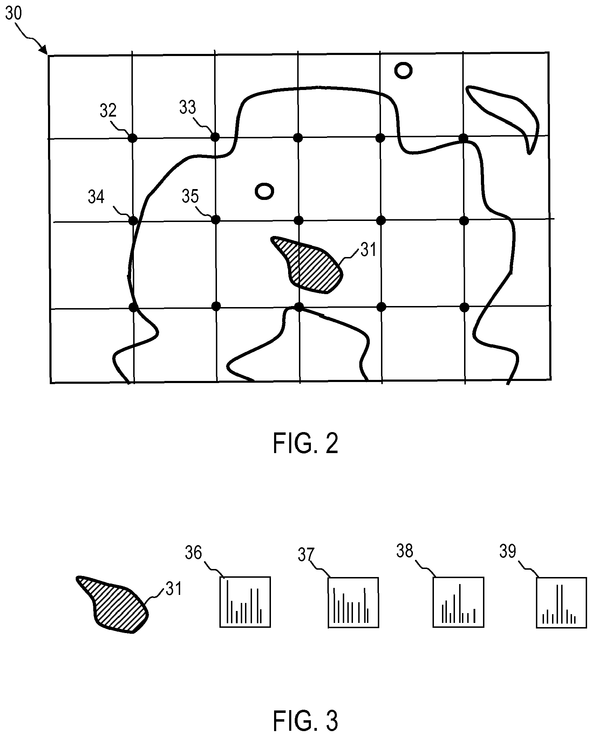

Wie unter Bezugnahme auf Figur 2 bis Figur 15 ausführlicher beschrieben wird, kann die integrierte Halbleiterschaltung 22 eingerichtet sein, um die Anwesenheit oder Abwesenheit bestimmter Raman-Peaks zu erkennen oder das spektrale Gewicht von Raman-Peaks zu bestimmen, die mit bösartigen Prostatatumoren zusammenhängen. Die integrierte Halbleiterschaltung 22 kann beispielsweise eingerichtet sein, um durch Auswertung des wenigstens einen Raman-Spektrums quantitativ zu ermitteln, ob und in welcher Menge Biomarker für einen bösartigen Prostatatumor vorhanden sind. Die integrierte Halbleiterschaltung 22 kann eingerichtet sein, um für eine Gewebeprobe örtliche Veränderungen und insbesondere örtliche Gradienten von Raman-Signalen zu bestimmen, die bösartigen Prostatatumore zugeordnet sind. Charakteristische ortsabhängige Veränderungen von Raman-Spektren, die einem aggressiven Prostatatumor zugeordnet sind, können in einer Datenbank hinterlegt sein. Die ortsabhängige Veränderung des Raman-Signals, für die in der Datenbank Informationen hinterlegt sind, kann eine Veränderung des Raman-Signals in Stroma der Prostata angeben.

Die Raman-Peaks, die von der integrierten Halbleiterschaltung 22 ausgewertet werden, können molekularen oder genetischen Biomarkern zugeordnet sein. Die Raman-Peaks, die von der integrierten Halbleiterschaltung 22 ausgewertet werden, können Protein-Biomarkern zugeordnet sein.

Beispiele für Biomarker zur Erkennung der Aggressivität von Prostatatumoren sind Carboxypeptidase A3 (CPA3), Annexin 3 (ANXA 3), Sarkosin und/oder PSA. Die Biomarker können ausgewählt sein aus der Gruppe bestehend aus AMACR, FOLH1 (PSMA), PTEN, PCA3, ERG3 und PSA. Mit Vorrichtungen und Verfahren kann ein Biomarker oder können mehrere Biomarker, die ausgewählt sind aus einer Gruppe bestehend aus AMACR, FOLH1 (PSMA), PTEN, PCA3, ERG3 und PSA, durch Raman-Spektroskopie erkannt werden.

Die Vorrichtung 1 kann eingerichtet sein, um die Anwesenheit und optional auch die räumliche Verteilung derartiger Biomarker zu bestimmen. Einige der Biomarker können nahe an einem bösartigen Prostatatumor stärker auftreten, während andere der Biomarker in einer Entfernung von einem bösartigen Prostatatumor stärker auftreten als in unmittelbarer Nähe des bösartigen Prostatatumors.

Die integrierte Halbleiterschaltung 22 kann erfasste Raman-Spektren auf unterschiedliche Weise verarbeiten. Beispielsweise können statistische Methoden, z.B. eine Hauptkomponentenanalyse, verwendet werden. Andere statistische Methode, beispielsweise eine hierarchische Clusteranalyse, können verwendet werden, um zu beurteilen, ob ein Raman-Spektrum in seiner Gesamtheit einem aggressiven oder einem nicht aggressiven Prostatatumor zuzuordnen ist. Zusätzlich oder alternativ können Raman-Spektren oder die ortsabhängige Veränderung wenigstens einzelner Raman-Peaks mit Referenzdaten verglichen werden, um zu bestimmen, ob ein aggressiver Prostatatumor vorhanden ist. Die Bestimmung des Gradienten ist nicht auf die Intensität einzelner Raman-Peaks beschränkt, sondern kann beispielsweise auch für den Abstand eines Datenpunkts in einem N-dimensionalen Datenraum einer Hauptkomponentenanalyse von dem Bereich in dem N-dimensionalen Datenraum, der einem aggressiven Prostatatumor zugeordnet ist, vorgenommen werden. Die entsprechende Verarbeitung kann von der integrierten Halbleiterschaltung 22 automatisch ausgeführt werden.

Die Auswerteeinrichtung 20 kann einen Speicher 23 umfassen, in dem die Referenzdaten 24 hinterlegt sind, die die integrierte Halbleiterschaltung 22 bei der Auswertung des Raman-Spektrums mit verwenden kann.

Die Auswerteeinrichtung 20 kann eine optische und/oder akustische Ausgabeeinheit 25 umfassen, über die abhängig von der Analyse des wenigstens einen Raman-Spektrums Information ausgegeben wird, die anzeigt, ob ein aggressiver Prostatatumor vorhanden ist. Es kann eine Beurteilung der Aggressivität des Prostatatumors ausgegeben werden. Es kann Information über ein Stadium des Prostatakarzinoms ausgegeben werden.

Auch wenn die Auswerteeinrichtung 20 und das Raman-Spektroskopiesystem 10 in Figur 1 schematisch als separate Einheiten dargestellt sind, können die Funktionen der Auswerteeinrichtung 20 auch in einem Gehäuse des Raman-Spektroskopiesystems 10 integriert sein. Das Raman-Spektroskopiesystem 10 und die Auswerteeinrichtung 20 können als mobile, insbesondere als tragbare Einheiten ausgestaltet sein.

Figur 2 zeigt schematisch eine Gewebeprobe 30. Die Gewebeprobe ist als Gewebeschnitt dargestellt. Die beschriebenen Techniken können aber auch an Gewebeproben anderer Geometrie verwendet werden, beispielsweise an mit Stanzbiopsie gewonnenen Biopsaten. Ein bösartiger Tumor 31, der nur schematisch dargestellt ist, kann vorhanden sein. Die Gewebeprobe 30 kann Zellen des Stroma der Prostata enthalten. Für die beschriebenen Auswertetechniken ist insbesondere nicht erforderlich, dass Tumorzellen selbst in der Gewebeprobe 30 enthalten sind.

An einer Mehrzahl von Bereichen, die als Punkte bzw. gefüllte Kreise dargestellt sind, kann jeweils wenigstens ein Raman-Spektrum erfasst werden. Zur Verbesserung der Statistik können an jedem der Punkte auch mehrere Raman-Spektren erfasst werden. Die Signalerfassung und Relativbewegung zwischen einem Objektträger und optischen Komponenten des Raman-Spektroskopiesystems kann von der Auswerteeinrichtung 20 automatisch gesteuert werden. Beispielsweise können an Mehreren voneinander getrennten kleinen Bereichen 32-35 jeweils Raman-Spektren erfasst werden.

Auch wenn in Figur 2 schematisch eine reguläre Anordnung von Punkten dargestellt ist, an denen die Raman-Spektren erfasst werden, kann die Erfassung auch an einer irregulären Anordnung von Punkten erfolgen. Es können unterschiedliche Muster von Punkten definiert werden, an denen die Raman-Spektroskopie jeweils ausgeführt werden soll. Wenigstens einige der Punkte 32-35 können auch benutzerdefiniert festlegbar sein. Die Auswerteeinrichtung 20 kann eine entsprechende Eingabeschnittstelle umfassen, mit der eine benutzerdefinierte Festlegung derjenigen Punkte ermöglicht wird, an denen jeweils ein Raman-Spektrum erfasst werden soll.

Die ortsaufgelöste Erfassung der Raman-Spektren kann in größeren Abständen erfolgen. Nach Ausführungsbeispielen kann die ortsaufgelöste Erfassung von wenigstens zwei Raman-Spektren aber auch an subzellulären Strukturen erfolgen. Es können wenigstens zwei Raman-Spektren an unterschiedlichen subzellulären Bereichen, beispielsweise für Zellkerne und Cytoplasma, in einer Gewebeprobe erfasst und von der Auswerteeinrichtung 20 ausgewertet werden.

Die erfassten Raman-Spektren können in unterschiedlicher Weise ausgewertet werden. Beispielsweise kann für jedes der Raman-Spektren eine statistische Analyse, z.B. eine Hauptkomponentenanalyse oder eine hierarchische Clusteranalyse, ausgeführt werden. Auf diese Weise kann eine Beurteilung erfolgen, ob ein Raman-Spektrum oder die Mehrzahl von erfassten Raman-Spektren einem aggressiven oder einem nicht aggressiven Prostatatumor zugeordnet sind. Eine Zuweisung einzelner Raman-Peaks bzw. bestimmter Spektren-Muster zu Biomarkern kann, muss jedoch nicht ausgeführt werden.

Die Auswerteeinrichtung 20 kann eingerichtet sein, um aus der Gesamtheit des Raman-Spektrums zu ermitteln, ob ein aggressiver oder nicht aggressiver Prostatatumor vorliegt. Mit einer statistischen Analyse, z.B. einer PCA oder Clustertechniken, kann ein Muster oder können mehrere Muster in dem Spektrum als Unterscheidungskriterium zur Unterscheidung aggressiver und nicht aggressiver Prostatatumore verwendet werden. Derartige Muster können durch die Lage mehrerer Raman-Peaks, deren Höhe, und/oder die Steilheit der Flankendefiniert werden.

Figur 3 zeigt beispielhaft die Auswertung von Raman-Spektren 36-39 zur Erkennung und optional zur Bestimmung der Aggressivität von Prostatatumoren. Die Raman-Spektren 36-39 können an unterschiedlichen Positionen erfasst werden. Die Lage relativ zum Tumor 31 kann vorab auch unbekannt sein. Abhängig von der Position zeigen sich Veränderungen der Raman-Spektren 36-39, aus denen auf das Vorliegen eines aggressiven oder nicht aggressiven Prostatatumors geschlossen werden kann.

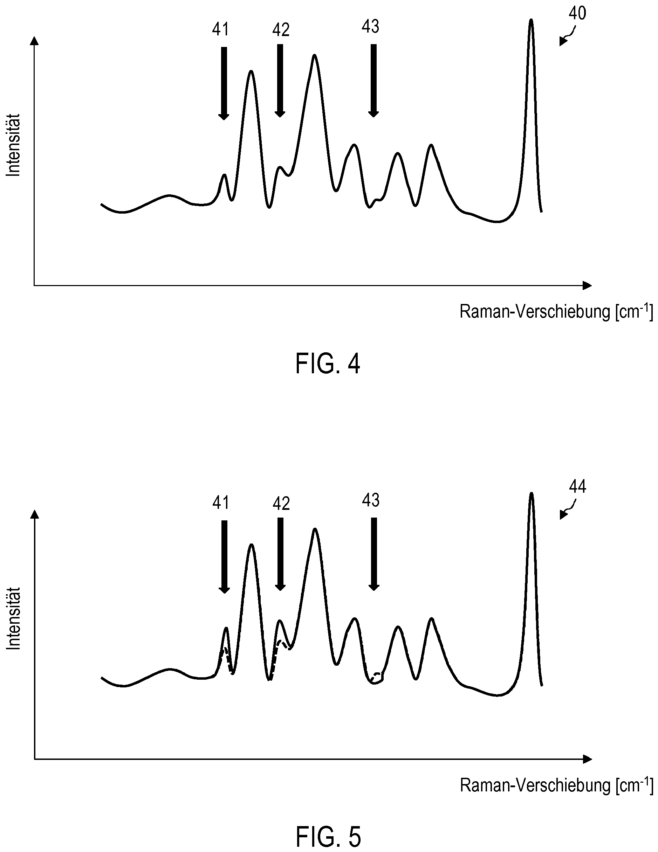

Figur 4 zeigt ein beispielhaftes Raman-Spektrum 40, das von der Vorrichtung 1 an der Probe 9 erfasst wurde. Beispielsweise kann die Vorrichtung 1 das Raman-Spektrum 40 an einem Punkt 33 der Gewebeprobe 30 erfassen.

Die Auswerteeinrichtung 20 kann automatisch einen Raman-Peak oder mehrere Raman-Peaks 41-43 erkennen, die für Prostatakrebs charakteristisch sind. Die Auswerteeinrichtung 20 kann eingerichtet sein, um einen für Prostatakrebs charakteristischen Wellenzahlenbereich oder mehrere für Prostatakrebs charakteristische Wellenzahlenbereiche mit Raman-Peaks 41-43 zu erkennen. Einer oder mehrere der Raman-Peaks 41-43 können einem Biomarker für aggressive Prostatatumore zugeordnet sein.

Die Auswerteeinrichtung 20 kann alternativ oder zusätzlich automatisch charakteristische Muster eines oder mehrerer Raman-Spektren 41-43 erkennen. Dabei können die Wellenzahlen, an denen Raman-Peaks liegen, die Peakhöhe, die Flankensteilheit, Abstände zwischen den Peaks und/oder Kombinationen von Peaks in einem oder mehreren Raman-Spektren 41-43 erkannt werden, um zu bestimmen, ob ein aggressiver oder ein nicht aggressiver Prostatatumor vorliegt und/oder um einen Prostatatumor zu erkennen.

Anhand der Anwesenheit und optional auch der Intensität des Raman-Signals bei den Raman-Peaks 41-43, die für Prostatakrebs charakteristisch sind, kann erkannt werden, ob Prostatakrebs vorliegt. Anhand der Intensität des Raman-Signals bei einem oder mehrerer der Raman-Peaks 41-43 kann die Auswerteeinrichtung 20 den Prostatakrebs beurteilen, um eine Aggressivität zu bestimmen.

Die in Figur 4 schematisch dargestellten Raman-Peaks müssen nicht individuell erkannt werden. Insbesondere kann die Auswerteeinrichtung 20 das Raman-Spektrum in seiner Gesamtheit einer statistischen Analyse unterziehen, um zu erkennen, ob das Raman-Spektrum einem aggressiven oder einem nicht aggressiven Prostatatumor zugeordnet ist.

Es können auch mehrere Raman-Spektren an der Probe 9 erfasst werden. Ein Vergleich der unterschiedlichen Raman-Spektren kann verwendet werden, um Aussagen darüber zu treffen, ob ein bösartiger Prostatatumor vorhanden ist, und/oder um die Aggressivität des bösartigen Prostatatumors zu beurteilen.

Figur 5 zeigt beispielhaft ein weiteres Raman-Spektrum 44, das an einer anderen Position der Probe 9 erfasst wird als das Raman-Spektrum 40. Das Raman-Spektrum 40 ist in Figur 5 mit durchbrochenen Linien zum Vergleich ebenfalls dargestellt.

Beispielsweise kann das weitere Raman-Spektrum 44 an einer Position 35 erfasst werden, die näher an dem Tumor liegt als die Position 33, an der das Raman-Spektrum 40 erfasst wurde. Auch wenn die Lage des Prostatatumors oder sogar seine Anwesenheit a priori nicht bekannt ist, kann anhand eines Vergleichs des weiteren Raman-Spektrums 44 an der Position 35 und des Raman-Spektrums 40 an der Position 33 bestimmt werden, ob ein bösartiger Prostatatumor vorhanden ist. Es kann auch bestimmt werden, in welcher Richtung sich der Tumor relativ zu den Positionen 33, 35 befindet.

Die Intensität des Raman-Signals und/oder das spektrale Gewicht der Raman-Peaks 41-43 kann in dem Raman-Spektrum 44 von der Intensität und/oder dem spektralen Gewicht in dem Raman-Spektrum 40 verschieden sein. Beispielsweise kann die Intensität des Signals an einem oder mehreren Raman-Peaks 41, 42 größer werden, wenn man sich der Position des bösartigen Prostatatumors nähert, wenn der entsprechende Biomarker in unmittelbarer Nähe des Prostatatumors stärker vorhanden ist als an entfernteren Positionen. Die Intensität des Signals an einem oder mehreren weiteren Raman-Peaks 43 kann kleiner werden, wenn man sich der Position des bösartigen Prostatatumors nähert, wenn der entsprechende Biomarker in unmittelbarer Nähe des Prostatatumors weniger stark vorhanden ist als an entfernteren Positionen.

Der Gradient der Raman-Peaks 41-43 muss nicht individuell bestimmt werden. Insbesondere kann die Auswerteeinrichtung 20 das Raman-Spektrum in seiner Gesamtheit einer statistischen Analyse unterziehen, um zu erkennen, ob das Raman-Spektrum einem aggressiven oder einem nicht aggressiven Prostatatumor zugeordnet ist. Wie noch ausführlicher beschrieben wird, kann beispielsweise eine Zuordnung von Raman-Spektren zu aggressiven oder nicht aggressiven Prostatatumoren anhand des Ergebnisses einer statistischen Analyse von der Vorrichtung automatisch vorgenommen werden, ohne dass hierfür die einzelnen Raman-Peaks 41-43 vorbekannt sein müssen.

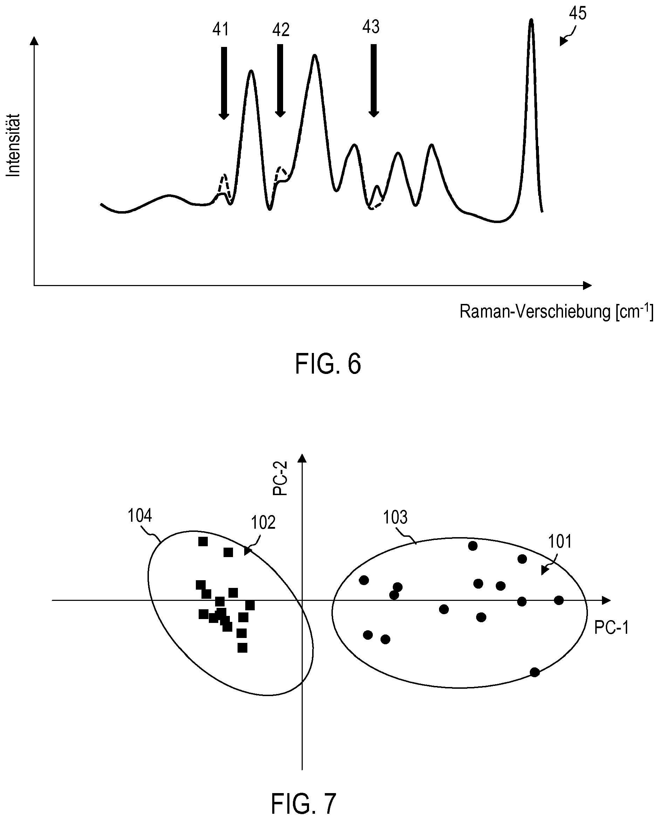

Figur 6 zeigt beispielhaft ein weiteres Raman-Spektrum 45, das an einer anderen Position der Probe 9 erfasst wird als das Raman-Spektrum 40. Das Raman-Spektrum 40 ist mit durchbrochenen Linien zum Vergleich ebenfalls dargestellt.

Beispielsweise kann das weitere Raman-Spektrum 45 an einer Position 32 erfasst werden, die weiter entfernt von dem Tumor liegt als die Position 33, an der das Raman-Spektrum 40 erfasst wurde. Auch wenn die Lage des Prostatatumors oder sogar seine Anwesenheit a priori nicht bekannt ist, kann anhand eines Vergleichs des weiteren Raman-Spektrums 45 an der Position 32 und des Raman-Spektrums 40 an der Position 33 bestimmt werden, ob ein bösartiger Prostatatumor vorhanden ist. Es kann auch bestimmt werden, in welcher Richtung sich der Tumor relativ zu den Positionen 33, 35 befindet.

Die Intensität des Raman-Signals und/oder das spektrale Gewicht der Raman-Peaks 41-43 kann in dem Raman-Spektrum 45 von der Intensität und/oder dem spektralen Gewicht in dem Raman-Spektrum 40 verschieden sein. Beispielsweise kann die Intensität des Signals an einem oder mehreren Raman-Peaks 41, 42 kleiner werden, wenn man sich von der Position des bösartigen Prostatatumors entfernt, wenn der entsprechende Biomarker in unmittelbarer Nähe des Prostatatumors stärker vorhanden ist als an entfernteren Positionen. Die Intensität des Signals an einem oder mehreren weiteren Raman-Peaks 43 kann größer werden, wenn man sich der Position des bösartigen Prostatatumors entfernt, wenn der entsprechende Biomarker in unmittelbarer Nähe des Prostatatumors weniger stark vorhanden ist als an entfernteren Positionen.

Der Gradient der Raman-Peaks 41-43 muss nicht individuell bestimmt werden. Insbesondere kann die Auswerteeinrichtung 20 das Raman-Spektrum in seiner Gesamtheit einer statistischen Analyse unterziehen, um zu erkennen, ob das Raman-Spektrum einem aggressiven oder einem nicht aggressiven Prostatatumor zugeordnet ist. Wie noch ausführlicher beschrieben wird, kann beispielsweise eine Zuordnung von Raman-Spektren zu aggressiven oder nicht aggressiven Prostatatumoren anhand des Ergebnisses einer statistischen Analyse von der Vorrichtung automatisch vorgenommen werden, ohne dass hierfür die einzelnen Raman-Peaks 41-43 vor Durchführung der Messung vorbekannt sein müssen.

Die Information mehrerer Raman-Spektren 40, 44, 45 kann kombiniert werden, um Information über die Anwesenheit eines bösartigen Prostatatumors oder seine Aggressivität zu erhalten.

Bei einigen Ausführungsbeispielen kann ein örtlicher Gradient der Intensität des Raman-Signals an einem oder mehreren der Raman-Peaks 41-43, die für einen bösartigen Prostatatumor charakteristisch sind, ermittelt werden. Dazu kann für mehrere Punkte jeweils rechnerisch der Gradient der Intensität oder des spektralen Gewichts des Raman-Peaks in einer, zwei oder drei Dimensionen bestimmt werden. Allgemein kann eine Bestimmung des Gradienten in n Dimensionen erfolgen, wobei n gleich eins oder größer als eins sein kann.

Der Gradient kann auch anhand des Ergebnisses einer weitergehenden Analyse der Raman-Spektren erfolgen. Beispielsweise kann durch eine statistische Auswertung jedes Raman-Spektrum einem Punkt in einem N-dimensionalen Datenraum zugeordnet werden, wobei N >> 1, beispielsweise N > 100 sein kann. Der N-dimensionale Datenraum kann der bei einer Hauptkomponentenanalyse von den verschiedenen Hauptkomponenten aufgespannte Datenraum sein. Aus Referenzspektren ist ermittelbar, in welchen Bereichen des N-dimensionalen Datenraums Raman-Spektren für aggressive Prostatatumore clusterartig angeordnet sind und in welchen anderen Bereichen des N-dimensionalen Datenraums Raman-Spektren für nicht aggressive Prostatatumore clusterartig angeordnet sind.

Die ortsabhängige Veränderung von Raman-Spektren kann beinhalten, dass ortsabhängig weitere Raman-Peaks auftauchen, die auf das Vorhandensein weiterer Biomoleküle abhängig von einem Abstand zum Tumor hinweisen und die Aggressivität oder Nichtaggressivität des Tumors anzeigen können. Auch können Raman-Peaks ortsabhängig (d.h. in einem ortsabhängigen Verlauf) verschwinden, woraus auch auf die Aggressivität oder Nichtaggressivität des Tumors Rückschlüsse gezogen werden können. Die ortsabhängige Veränderung von Raman-Spektren kann beinhalten, dass sich der Punkt, der das entsprechende Raman-Spektrum in einem N-dimensionalen Datenraum, wobei N >> 1, beispielsweise N > 100 sein kann, verschiebt. Der N-dimensionale Datenraum kann der bei einer Hauptkomponentenanalyse von den verschiedenen Hauptkomponenten aufgespannte Datenraum sein.

Um zu erkennen, ob ein Raman-Spektrum einen aggressiven oder einen nicht aggressiven Prostatatumor anzeigt, kann die Auswerteeinrichtung 20 somit eingerichtet sein, um eine Hauptkomponentenanalyse, eine Clusteranalyse oder eine andere statistische Analyse wenigstens eines Raman-Spektrums auszuführen. Die Auswerteeinrichtung 20 kann eingerichtet sein, um durch die statistische Analyse quantitativ zu erkennen, ob das Raman-Spektrum zu einem Cluster oder einem Bereich des bei der Hauptkomponentenanalyse aufgespannten Datenraums gehört, die den Raman-Spektren aggressiver Prostatatumore zugeordnet sind. Die Auswerteeinrichtung 20 kann eingerichtet sein, um durch die statistische Analyse quantitativ zu erkennen, ob das Raman-Spektrum zu einem Cluster oder einem Bereich des bei der Hauptkomponentenanalyse aufgespannten Datenraums gehört, die den Raman-Spektren nicht aggressiver Prostatatumore zugeordnet sind.

Figur 7 zeigt die Ergebnisse einer Hauptkomponentenanalyse für aggressive und nicht aggressive Prostatatumore. Zur Hauptkomponentenanalyse können die jeweils erfassten Raman-Spektren an einer Mehrzahl N >> 1, beispielsweise N > 100, von Raman-Wellenzahlen ausgewertet werden. Der N-dimensionale Vektor, der den Raman-Intensitäten an den N Raman-Wellenzahlen entspricht, definiert einen Punkt in einem N-dimensionalen Datenraum.

Durch die Hauptkomponentenanalyse (PCA, "Principal Component Analysis") wird eine Koordinatentransformation in dem N-dimensionalen Datenraum derart ermittelt, dass sich die unterschiedlichen Cluster oder Gruppen von Raman-Spektren in dem transformierten Koordinatensystem entlang einer oder mehrerer der Koordinatenachsen deutlich unterscheiden. Diese Koordinatenachsen definieren die Hauptkomponenten. Die erste Hauptkomponente PC-1 definiert dabei typischerweise die Achse mit den deutlichsten Unterschieden zwischen den unterschiedlichen Gruppen von Raman-Spektren.

Figur 7 zeigt, wie sich die Datenpunkte 101, die Raman-Spektren aggressiver Prostatatumore entsprechen, von Datenpunkten 102, die Raman-Spektren nicht aggressiver Prostatatumore entsprechen, entlang der Hauptkomponentenachse PC-1 trennen.

Durch diese bei der Hauptkomponentenanalyse auftretende Trennung kann aus einem Raman-Spektrum einer Probe oder aus mehreren Raman-Spektren der Probe ermittelt werden, ob diese einem aggressiven oder nicht aggressiven Prostatatumor zugeordnet sind. Dazu kann jedes Raman-Spektrum jeweils bei den N Raman-Wellenzahlen abgetastet und dann in die Ebene oder in den Raum projiziert werden, der durch die Hauptkomponentenachsen der niedrigsten Ordnungen aufgespannt ist. Beispielsweise kann anhand der PC-1-Komponente, d.h. der ersten Hauptkomponente, die die deutlichsten Unterschiede zwischen den Raman-Spektren aggressiver und nicht aggressiver Prostatatumore zeigt, ermittelt werden, ob ein aggressiver oder ein nicht aggressiver Prostatatumor vorliegt. Alternativ oder zusätzlich kann anhand der zweiten Hauptkomponente PC-2 oder einer anderen niedrigen Hauptkomponente ermittelt werden, ob ein aggressiver oder ein nicht aggressiver Prostatatumor vorliegt.

Es sollte beachtet werden, dass die Bestimmung, ob das Raman-Spektrum für einen aggressiven oder einen nicht aggressiven Prostatatumor charakteristisch ist, nicht anhand einzelner Raman-Peaks, sondern anhand einer Mehrzahl von über das Raman-Spektren gleichmäßig oder ungleichmäßig verteilter Raman-Intensitäten bei einer Mehrzahl von Raman-Wellenzahlen erfolgen kann. Mit der Hauptkomponentenanalyse oder anderen statistischen Methoden wie hierarchischen oder nicht hierarchischen Clusteranalysen kann somit genutzt werden, dass das Raman-Spektrum als Ganzes Charakteristika aufweist, die für einen aggressiven oder nicht aggressiven Prostatatumor kennzeichnend sind und die somit als ein "photonischer Fingerabdruck" dienen.

Durch eine statistische Analyse, wie die Hauptkomponentenanalyse oder die später noch ausführlicher diskutierte Clusteranalyse, kann ermittelt werden, ob das in dem Raman-Spektrum enthaltene Muster von Raman-Peaks für einen aggressiven oder einen nicht aggressiven Prostatatumor charakteristisch ist. Alternativ oder zusätzlich kann ermittelt werden, ob das in dem Raman-Spektrum enthaltene Muster von Raman-Peaks für Vorhandensein eines Prostatatumors charakteristisch ist.

Das Muster in einem Raman-Spektrum kann definiert werden durch eine oder mehrere Kenngrößen, die ausgewählt sind aus der Gruppe bestehend aus: den Wellenzahlen, an denen Raman-Peaks liegen, den Peakhöhen, der Flankensteilheit der Peaks, den Abständen zwischen den Peaks und/oder Kombinationen von Peaks in einem oder mehreren Raman-Spektren.

Zur Auswertung einer oder mehrerer an einer Probe erfassten Raman-Spektren kann jeweils ermittelt werden, ob diese bei einer Hauptkomponentenanalyse in einem Bereich 103, der aggressiven Prostatatumoren zugeordnet ist, oder ein einem anderen Bereich 104, der nicht aggressiven Prostatatumoren zugeordnet ist, im Datenraum angeordnet sind.

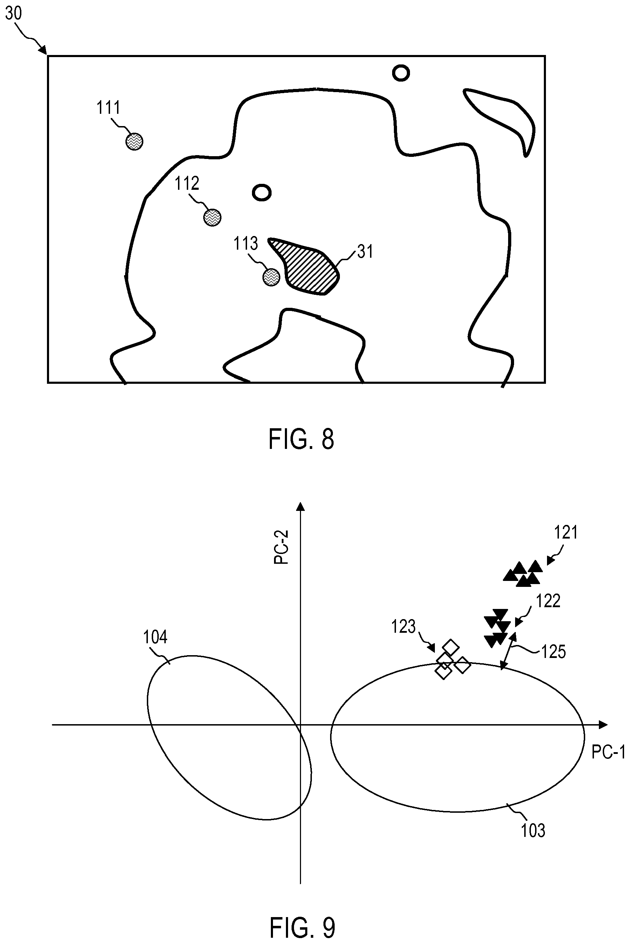

Figur 8 zeigt eine Probe 30 mit einem Prostatatumor 31. In mehreren Regionen 111, 112, 113 werden zur Auswertung der Probe 30 von der Vorrichtung 1 jeweils ein oder vorteilhaft mehrere Raman-Spektren erfasst. Eine, mehrere oder alle der Regionen 111, 112, 113 können auch im Stroma der Prostata liegen.

Die jeweils erfassten Raman-Spektren können von der Auswerteeinrichtung der Vorrichtung 1 einer statistischen Analyse unterzogen werden. Beispielsweise können die Raman-Spektren an einer Mehrzahl N von Raman-Wellenzahlen ausgewertet werden. Die Vektoren, die die N Raman-Intensitäten an den N Raman-Wellenzahlen enthalten, wobei $N \gg 1$, können durch eine Koordinatentransformation in den anhand von Referenzspektren aggressiver und nicht aggressiver Prostatatumore definierten Datenraum mit Hauptkomponentenachsen projiziert werden, wobei die niedrigste oder die niedrigsten Hauptkomponenten die deutlichste Trennung zwischen aggressiven und nicht aggressiven Prostatatumoren zeigen.

Figur 9 zeigt beispielhaft die Ergebnisse einer derartigen Auswertung, die von der Vorrichtung 1 vorgenommen wird.

Datenpunkte 121, die im Datenraum der Hauptkomponentenanalyse für in einer Region 111 erfasste Raman-Spektren gewonnen wurden, müssen nicht notwendig in oder unmittelbar an einem der Bereiche 103, 104 liegen, die aggressivem und nicht aggressivem Prostatatumorgewebe zugeordnet sind. Anhand einer Nähe oder einem Überlapp mit einem der Bereiche 103, 104 kann jedoch ein Hinweis auf das Vorliegen eines aggressiven oder nicht aggressiven Prostatatumors gewonnen werden, selbst wenn die Region 111 im Stroma angeordnet ist.

Datenpunkte 122, die im Datenraum der Hauptkomponentenanalyse für in einer Region 112 erfasste Raman-Spektren gewonnen wurden, müssen nicht notwendig in oder unmittelbar an einem der Bereiche 103, 104 liegen, die aggressivem und nicht aggressivem Prostatatumorgewebe zugeordnet sind. Anhand einer Nähe oder einem Überlapp mit einem der Bereiche 103, 104 kann jedoch ein Hinweis auf das Vorliegen eines aggressiven oder nicht aggressiven Prostatatumors gewonnen werden, selbst wenn die Region 112 im Stroma angeordnet ist. Aus der Tatsache, dass die Datenpunkte 122 einen Abstand 125 vom Bereich 103 aufweisen, der kleiner ist als ein Abstand der Datenpunkte 121 vom Bereich 103, kann geschlossen werden, dass die Region 112 näher an einem aggressiven Prostatatumor liegt als die Region 111. Dies kann zur systematischen Bestimmung der Lage des Prostatatumors verwendet werden, selbst wenn die Raman-Spektren nicht an einer Prostatatumorzelle selbst erfasst wurden.

Die Abstände zwischen den Datenpunkten 121, 122 und dem Bereich 103 können auf unterschiedliche Weise bestimmt werden. Beispielsweise kann der Schwerpunkt der Datenpunkte 121 bzw. 122 bestimmt und dann dessen Abstand vom Schwerpunkt des Bereichs 103 ermittelt werden. Es kann der Schwerpunkt der Datenpunkte 121 bzw. 122 bestimmt und dann dessen Abstand vom Rand des Bereichs 103 ermittelt werden. Es können die Abstände für jeden der Datenpunkte 121 bzw. 122 vom Schwerpunkt oder Rand des Bereichs 103 ermittelt werden, wobei anschließend eine Mittelung der Abstände vorgenommen werden kann. Entsprechend können Abstände auch vom Bereich 104, der nicht aggressiven Prostatatumoren zugeordnet ist, bestimmt werden. Der Abstand von einem Rand eines der Bereiche 103, 104 ist dabei jeweils definiert als das Minimum der Entfernung in dem Datenraum zwischen dem entsprechenden Datenpunkt und allen auf dem Rand des entsprechenden Bereiches 103, 104 liegenden Punkten im Datenraum.

Datenpunkte 123, die im Datenraum der Hauptkomponentenanalyse für in einer Region 113 erfasste Raman-Spektren gewonnen wurden, können in oder unmittelbar an einem der Bereiche 103, 104 liegen, die aggressivem und nicht aggressivem Prostatatumorgewebe zugeordnet sind. Durch den Überlapp kann erkannt werden, dass die Region 113 nahe an oder überlappend mit einem aggressiven Prostatatumor angeordnet ist. Aus der Tatsache, dass die Datenpunkte 123 einen Abstand vom Bereich 103 aufweisen, der kleiner ist als ein Abstand der Datenpunkte 121 vom Bereich 103 und als ein Abstand 125 der Datenpunkte 122 vom Bereich 103, kann geschlossen werden, dass die Region 113 näher an einem aggressiven Prostatatumor liegt als die Regionen 111 und 112. Dies kann zur systematischen Bestimmung der Lage des Prostatatumors verwendet werden, selbst wenn die Raman-Spektren nicht an einer Prostatatumorzelle selbst erfasst wurden.

Alternativ oder zusätzlich zur Hauptkomponentenanalyse können auch hierarchische oder nicht hierarchische Clusteranalysen verwendet werden, um jeweils zu ermitteln, ob ein Raman-Spektrum in seiner Gesamtheit eine größere Ähnlichkeit mit den Raman-Spektren von Referenzproben, die aggressive Prostatatumore enthalten, oder mit den Raman-Spektren von Referenzproben, die nicht aggressive Prostatatumore enthalten, aufweisen.

Bei derartigen Clusteranalysen können Spektren anhand eines Ähnlichkeitsmaßes zusammengruppiert oder geclustert werden. Unterschiedliche Ähnlichkeitsmaße können verwendet werden. Beispielsweise kann eine Cosinus-Entfernung verwendet werden, um eine Abstandsmetrik für Raman-Spektren zu definieren.

Bei hierarchischen Clusteranalysen kann ein Baum von Raman-Spektren aufgebaut werden, bei dem eine Zuordnung von Raman-Spektren zu gleichen oder unterschiedlichen Knoten jeweils von dem Ähnlichkeitsmaß abhängt.

Bei hierarchischen Clusteranalysen können nach dem Aufbauen eines Baumes mit Raman-Spektren dieser in zwei oder mehr Teilbäume unterteilt werden. Diese Teilbäume können beispielsweise aggressiven Prostatatumoren, nicht aggressiven Prostatatumoren oder Gewebe ohne Tumor zugeordnet sein.

Der Aufbau des Baums von Referenz-Spektren muss nicht bei jeder Auswertung von erfassten Raman-Spektren erfolgen. Vielmehr kann anhand einer Mehrzahl von Referenzspektren, die aggressiven oder nicht aggressiven Prostatatumoren zugeordnet wurden, der entsprechende Baum von Raman-Spektren aufgebaut werden. Bei einer Auswertung eines oder mehrerer an einer Probe erfassten Raman-Spektren kann jeweils ermittelt werden, in welchen Teilbaum oder in welche Knoten die entsprechenden Raman-Spektren anhand des Abstandsmaßes einzuordnen sind. Auf diese Weise kann das Raman-Spektrum, das an der Probe erfasst wurde, einem aggressiven oder nicht aggressiven Prostatatumor oder einer Probe, die keinen Prostatatumor aufweist, zugeordnet werden.

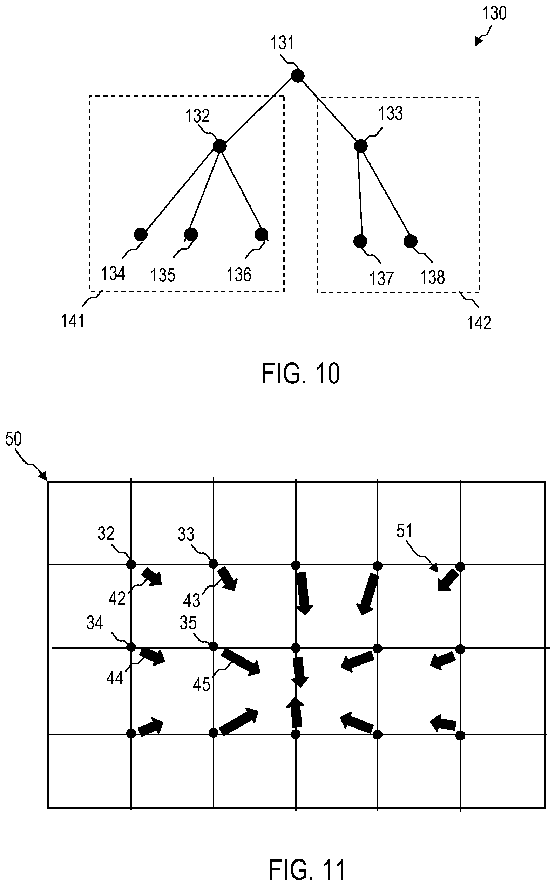

Figur 10 veranschaulicht schematisch die Funktionsweise der Auswerteeinrichtung der Vorrichtung 1 bei einer derartigen statistischen Auswertung, die eine Clusteranalyse umfasst.

Durch Auswertung einer Mehrzahl von Referenzspektren wird entweder von der Auswerteeinrichtung der Vorrichtung 1 selbst oder auch fern von der Vorrichtung 1 ein Baum 130 von Raman-Spektren aufgebaut. Der Baum 130 enthält eine Mehrzahl von Knoten 131-138. Raman-Spektren werden den Knoten 131-138 basierend auf einem Ähnlichkeitsmaß, das beispielsweise auf einer Cosinus-Entfernung beruhen kann, zugeordnet.

Der Baum 130 kann einen ersten Teilbaum 141 aufweisen, der aggressiven Prostatatumoren zugeordnet ist. Der Baum 130 kann einen zweiten Teilbaum 142 aufweisen, der nicht aggressiven Prostatatumoren zugeordnet ist. Bei der Auswertung eines Raman-Spektrums oder mehrerer Raman-Spektren, die an einer Probe 30 erfasst wurden, kann die Auswerteeinrichtung 20 der Vorrichtung 1 jeweils anhand des Abstandsmaßes berechnen, welchem der Blattknoten 134-138 und/oder inneren Knoten 132, 133 des Baumes 130 das entsprechende Raman-Spektrum zuzuordnen ist. Auf diese Weise können Raman-Spektren auch ohne a priori Kenntnis einzelner relevanter Wellenzahlen aggressiven oder nicht aggressiven Prostatatumoren zugeordnet werden. Wenigstens einer der unterschiedlichen Blattknoten 134-138 kann beispielsweise Raman-Spektren zugeordnet sein, die am Stroma einer Prostata mit einem aggressiven Prostatatumor erfasst wurden. Wenigstens ein weiterer der unterschiedlichen Blattknoten 134-138 kann beispielsweise Raman-Spektren zugeordnet sein, die am Stroma einer Prostata mit einem nicht aggressiven Prostatatumor erfasst wurden.

Die unter Bezugnahme auf Figur 7 und 9, 10 beschriebenen Techniken können bei Proben angewandt werden, die Gewebeschnitte oder Stanzbiopsate sind. Figur 11 zeigt beispielhaft die Bestimmung eines Gradientenfelds 50. Das Gradientenfeld 50 gibt für mehrere Positionen der Gewebeprobe an, wie stark und optional in welcher Richtung sich die Intensität eines Raman-Peaks 41-43 oder mehrerer Raman-Peaks 41-43 ändert, die für aggressive Prostatatumore charakteristisch sind. Die Mehrzahl von Gradienten 51 können jeweils eine Information über die Stärke der Abnahme oder Zunahme der Intensität des Raman-Peaks 41-43 aufweisen, die in Figur 11 durch die Länge der entsprechenden Pfeile dargestellt ist. Die Mehrzahl von Gradienten 51 können jeweils eine Information über die Richtung der Abnahme oder Zunahme der Intensität des Raman-Peaks 41-43 aufweisen, die in Figur 11 durch die Richtung der entsprechenden Pfeile dargestellt ist.

Die Gradienten 42-45, die beispielhaft dargestellt sind, können rechnerisch aus der Intensität oder dem spektralen Gewicht von Raman-Peaks 41-43 für Biomarker des bösartigen Prostatatumors bestimmt werden. Wie in Figur 11 schematisch dargestellt ist, geben die Gradienten Information über die Lage des Tumors.

Die Gradienten 51 müssen nicht anhand der Intensitäten der Raman-Peaks 41-43 ermittelt werden. Beispielsweise können die Gradienten 51 auch für den Abstand 125 ermittelt werden, den Datenpunkte bei einer statistischen Analyse von den Bereichen 103, 104 im Datenraum aufweisen, die aggressiven oder nicht aggressiven Prostatatumoren zugeordnet sind.

Verschiedene Techniken können eingesetzt werden, um ortsaufgelöst den Gradienten der Intensität oder des Spektralen Gewichts von Raman-Peaks zu ermitteln. Beispielsweise kann für einen Bereich mit Koordinaten (x, y) der Gradient 51 gemäß

$$\vec{g}(x,y) = \begin{pmatrix} [(I(x+\Delta x, y) - I(x,y))] / \Delta x \\ [(I(x, y+\Delta y) - I(x,y))] / \Delta y \end{pmatrix} \qquad (1)$$

bestimmt werden, wobei beispielhaft eine Datenerfassung in zwei Dimensionen angenommen wurde. In Gleichung (1) bezeichnet g den ermittelten Gradienten. Dabei bezeichnet I(x,y) die Intensität oder das spektrale Gewicht eines für einen aggressiven oder nicht aggressiven Prostatatumor charakteristischen Raman-Peaks bei Koordinaten (x, y). I(x+Δx, y) bezeichnet die Intensität oder das spektrale Gewicht desselben Raman-Peaks bei Koordinaten (x+Δx, y). I(x, y+Δy) bezeichnet die Intensität oder das spektrale Gewicht desselben Raman-Peaks bei Koordinaten (x, y+Δy).

Für die Erfassung von Raman-Spektren auf einem regulären, beispielsweise rechteckigen Netz, wie sie in Figur 30 beispielhaft dargestellt ist, kann der Gradient ermittelt werden als

$$\vec{g}(x,y) = \begin{pmatrix} [(I(x+a_x,y)-I(x-a_x,y)]/[2a_x] \\ [(I(x,y+a_y)-I(x,y-a_y)]/[2a_y] \end{pmatrix} \qquad (2)$$

wobei $a_x$ der Abstand zwischen benachbarten Datenerfassungspunkten entlang einer Koordinatenachse und $a_y$ der Abstand zwischen benachbarten Datenerfassungspunkten entlang einer dazu orthogonalen weiteren Koordinatenachse ist.

Bei einer Bestimmung der Gradienten anhand der Ergebnisse einer statistischen Analyse, z.B. einer Hauptkomponentenanalyse, können die entsprechenden Gleichungen (1) und (2) ebenfalls verwendet werden, wobei anstelle von I(x, y) jeweils der Abstand 125 des oder der Raman-Spektren, die an der entsprechenden Position erfasst wurden, im Datenraum der Hauptkomponentenanalyse eingesetzt werden kann.

Die Lage und auch die Aggressivität eines bösartigen Prostatatumors können sogar dann bestimmt werden, wenn keine Krebszellen in der Probe 9 selbst vorhanden sind. Dazu wird das Vorhandensein oder optional auch die räumliche Veränderung von Raman-Peaks oder der Ergebnisse der statistischen Analyse mehrerer Raman-Spektren als Funktion des Abstandes von dem Prostatatumor verwendet.

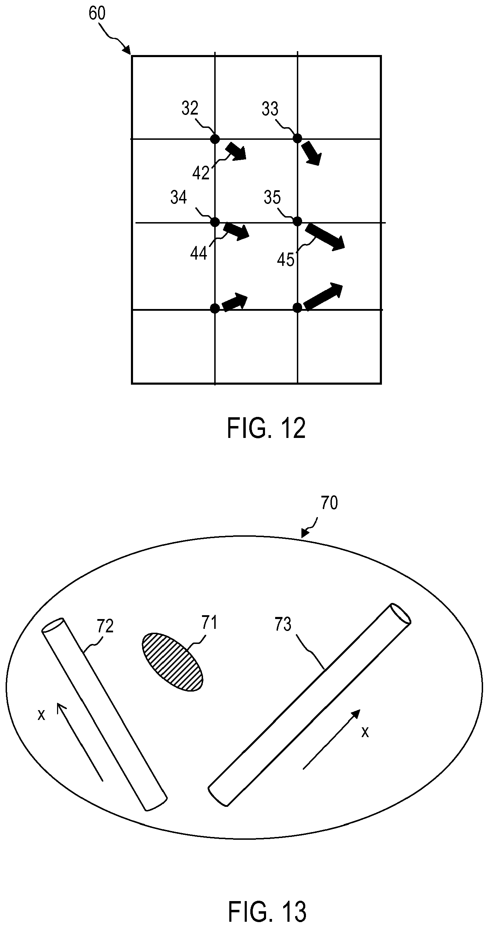

Figur 12 zeigt schematisch ein Gradientenfeld 60, das von der Auswerteeinrichtung 20 automatisch ermittelt wird und das die ortsabhängige Veränderung der Intensität oder des spektralen Gewichts von Raman-Peaks 41-43 oder der Abstände 125 vom Bereich 103 angibt, die für aggressive Prostatatumore charakteristisch sind. Auch wenn der Tumor nicht in der Probe enthalten ist, kann aus dem Verlauf der örtlichen Änderung der Intensität oder des spektralen Gewichts der Raman-Peaks 41-43 abgeschätzt werden, wo der Tumor liegt. Alternativ oder zusätzlich kann die Veränderung der Intensität oder des spektralen Gewichts von Raman-Peaks, die aus der Probe ermittelt wird, auch so extrapoliert werden, dass die Intensität oder das spektrale Gewicht des entsprechenden Raman-Peaks an dem nicht mehr von der Gewebeprobe erfassten Position des Prostatatumors abgeschätzt werden kann. Dies kann zur Beurteilung des Prostatatumors und/oder zur Stadienbestimmung eingesetzt werden.

Ähnliche Methoden können auch zur Untersuchung von Biopsaten, die beispielsweise mit Stanzbiopsie gewonnen werden, eingesetzt werden.

Figur 13 ist eine Darstellung 80, die die Lage von Biopsaten 72, 73 veranschaulicht, wie sie mit Stanzbiopsie gewonnen werden. Die Biopsate 72, 73 können Zellen eines bösartigen Prostatatumors 71 enthalten. Die Biopsate 72, 73 können jedoch auch so liegen, dass sie keine Zellen des bösartigen Prostatatumors 71 enthalten. Die Biopsate 72, 73 können Zellen des Stroma der Prostata enthalten.

Die Auswerteeinrichtung 20 kann an mehreren Positionen entlang den Biopsaten 72, 73 jeweils Raman-Spektren erfassen. Aus der Anwesenheit von Raman-Peaks, die Prostatakrebs zugeordnet sind, kann auf das Vorliegen eines bösartigen Prostatatumors geschlossen werden. Alternativ oder zusätzlich kann aus der örtliche Veränderung von Raman-Peaks, die Prostatakrebs zugeordnet sind, zusätzliche Information gewonnen werden. Beispielsweise kann aus der örtlichen Veränderung der Intensität und/oder des spektralen Gewichts von Raman-Peaks für Biomarker, die den bösartigen Prostatatumor anzeigen, auf die Aggressivität des Prostatatumors geschlossen werden.

Eine Erkennung der Aggressivität des Prostatatumors muss nicht anhand einzelner Raman-Peaks erfolgen, sondern kann auch anhand einer statistischen Analyse eines oder mehrerer Raman-Spektren insgesamt erfolgen. A priori Kenntnis über die Zuordnung von Raman-Wellenzahlen und Biomarkern ist nicht erforderlich.

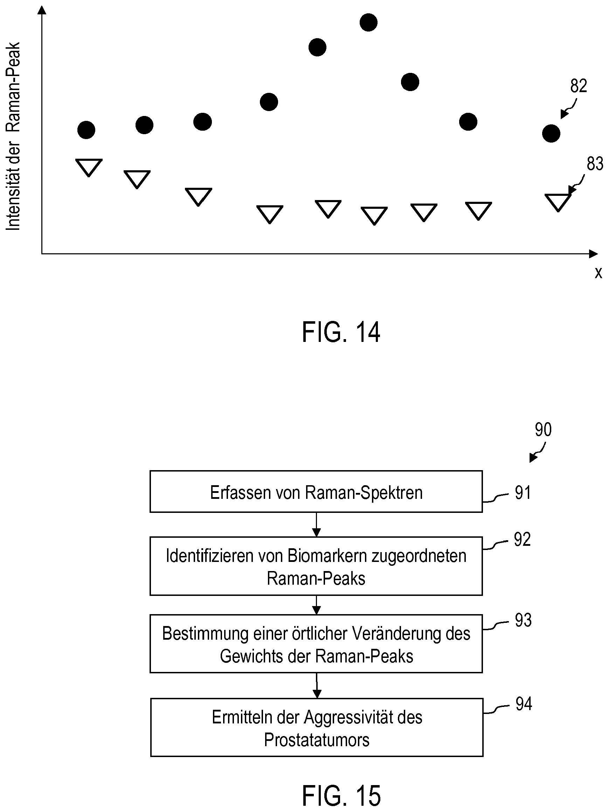

Figur 14 zeigt beispielhaft die Intensität eines Raman-Peaks, der beispielsweise ein Raman-Peak eines genetischen oder molekularen Biomarkers für Prostatakrebs sein kann, als Funktion des Ortes für das Biopsat 72 (Daten 82) und für das Biopsat 73 (Daten 83). Die dargestellte räumliche Veränderung der Intensität kann ausgewertet werden, um zu bestimmen, in welcher Menge der entsprechende Biomarker beispielsweise am Prostatatumor selbst vorhanden ist. Die Aggressivität des bösartigen Prostatatumors kann rechnerisch bestimmt werden.

Die elektronische Auswerteeinrichtung kann eingerichtet sein, um anhand des erfassten Raman-Spektrums, beispielsweise anhand eines charakteristischen Musters des Raman-Spektrums bei einer oder mehreren Wellenzahlen oder Wellenzahlbereichen, die Aggressivität des Prostatatumors zu bestimmen.

Eine Erkennung der Aggressivität des Prostatatumors muss nicht anhand einzelner Raman-Peaks erfolgen, sondern kann auch anhand einer statistischen Analyse eines oder mehrerer Raman-Spektren insgesamt erfolgen. A priori Kenntnis über die Zuordnung von Raman-Wellenzahlen und Biomarkern ist nicht erforderlich.

Auf diese Weise werden ein oder mehrere durch ein Raman-Spektrum definierte Muster in der Lage, Höhe, Flankensteilheit und Kombination mehrerer Raman-Peaks verwendet, um Aussagen über das Vorhandensein und/oder die Aggressivität eines Prostatatumors zu treffen. Das Muster in einem Raman-Spektrum kann definiert werden durch eine oder mehrere Kenngrößen, die ausgewählt sind aus der Gruppe bestehend aus: den Wellenzahlen, an denen Raman-Peaks liegen, den Peakhöhen, der Flankensteilheit der Peaks, den Abständen zwischen den Peaks und/oder Kombinationen von Peaks in einem oder mehreren Raman-Spektren.

Figur 15 ist ein Flussdiagramm eines Verfahrens 90 nach einem Ausführungsbeispiel.

Bei Schritt 91 wird wenigstens ein Raman-Spektrum der Probe 9 erfasst. Die Lichtquelle 11 wird so gesteuert, dass ein Anregungsstrahl 17 erzeugt wird. Es können auch mehrere Raman-Spektren erfasst werden. Beispielsweise können mehrere Raman-Spektren an unterschiedlichen Positionen derselben Probe oder an unterschiedlichen Proben erfasst werden, um einen bösartigen Prostatatumor zu erkennen.

Bei Schritt 92 wertet die Auswerteeinrichtung 20 das erfasste Raman-Spektrum aus. Dabei kann die Auswerteeinrichtung 20 wenigstens einen Raman-Peak erkennen, der für einen Biomarker charakteristisch ist, der Prostatakrebs anzeigt.

Bei Schritt 93 kann optional eine räumliche Veränderung wenigstens eines Raman-Peaks berechnet werden, der für einen Biomarker charakteristisch ist, der Prostatakrebs anzeigt

Bei Schritt 94 wird abhängig von der Auswertung des wenigstens einen Raman-Spektrums automatisch bestimmt, ob ein bösartiger Prostatatumor vorhanden ist. Es kann eine Aggressivität und/oder ein Stadium des Prostatatumors automatisch bestimmt werden.

Bei Vorrichtungen und Verfahren nach den beschriebenen Ausführungsbeispielen kann die Auswertung von Raman-Spektren jeweils einen Vergleich mit Raman-Peaks von Biomarkern umfassen, die aggressive oder nicht aggressive Prostatatumore anzeigen.

Mit Vorrichtungen und Verfahren kann ein Biomarker oder können mehrere Biomarker, die ausgewählt sind aus einer Gruppe bestehend aus AMACR, FOLH1 (PSMA), PTEN, PCA3, ERG3 und PSA, durch Raman-Spektroskopie erkannt werden. Es kann eine ortsaufgelöste Bestimmung der Veränderung der entsprechenden Raman-Peaks erfolgen, um den Prostatatumor zu erkennen und optional zu bestimmen, oder der Prostatakrebs aggressiv oder nicht aggressiv ist. Alternativ oder zusätzlich kann auch die Reaktion von Prostatazellen, Stromazellen oder anderen Bestandteilen der Probe auf von einem Prostatatumor erzeugte Stoffe erkannt werden. Auf diese Weise kann auf das Vorhandensein eines Prostatatumors geschlossen werden und/oder eine Unterscheidung aggressiver und nicht aggressiver Prostatatumore vorgenommen werden.

Vorrichtungen und Verfahren nach Ausführungsbeispielen können allgemein zur quantitativen Untersuchung von Proben zur Erkennung von Prostatatumoren eingesetzt werden. Die Vorrichtungen und Verfahren können insbesondere zur Untersuchung von zuvor gewonnenen Proben eingesetzt werden, wobei die Gewinnung der Proben nicht von den Verfahren zur Untersuchung umfasst wird. Der Schutzumfang wird durch die folgenden Patentansprüche bestimmt.

**Patentansprüche**

1. Vorrichtung zum Erkennen eines Prostatatumors, umfassend

   ein Raman-Spektroskopiesystem (10) zum Erfassen wenigstens eines Raman-Spektrums (36-39; 40; 44; 45) einer Gewebeprobe (9), und
   eine elektronische Auswerteeinrichtung (20), die eingerichtet ist, um abhängig von einer Auswertung des wenigstens einen Raman-Spektrum (36-39; 40; 44; 45) den Prostatatumor zu erkennen,
   wobei die Vorrichtung eingerichtet ist, um an einer Mehrzahl von Positionen (32-35) der Gewebeprobe (9) jeweils wenigstens ein Raman-Spektrum (36-39; 40; 44; 45) zu erfassen, und
   wobei die elektronische Auswerteeinrichtung (20) eingerichtet ist, um die an der Mehrzahl von Positionen erfassten Raman-Spektren (36-39; 40; 44; 45) auszuwerten, um durch die Auswertung der an der Mehrzahl von Positionen erfassten Raman-Spektren (36-39; 40; 44; 45) ein Gradientenfeld zu bestimmen, um eine Position des Prostatatumors in der Gewebeprobe (9) oder relativ zu der Gewebeprobe (9) zu ermitteln, wobei das Gradientenfeld für die an der Mehrzahl von Positionen (32-35) erfassten Raman-Spektren angibt, wie stark und in welcher Richtung sich eine Intensität eines Raman-Peaks oder mehrerer Raman-Peaks ändert,
   wobei die elektronische Auswerteeinrichtung (20) eingerichtet ist, um durch eine statistische Auswertung der an der Mehrzahl von Positionen erfassten Raman-Spektren (36-39; 40; 44; 45) zu bestimmen, ob ein aggressiver oder nicht aggressiver Prostatatumor vorliegt.

2. Vorrichtung nach Anspruch 1,
   wobei die elektronische Auswerteeinrichtung (20) eingerichtet ist, um durch eine Hauptkomponentenanalyse oder eine Clusteranalyse der an der Mehrzahl von Positionen erfassten Raman-Spektren (36-39; 40; 44; 45) zu bestimmen, ob ein aggressiver oder nicht aggressiver Prostatatumor vorliegt.

3. Vorrichtung nach einem der vorhergehenden Ansprüche,
wobei die elektronische Auswerteeinrichtung (20) eingerichtet ist, um durch die Auswertung der an der Mehrzahl von Positionen erfassten Raman-Spektren (36-39; 40; 44; 45) Raman-Peaks und charakteristische Muster in den an der Mehrzahl von Positionen erfassten Raman-Spektren (36-39; 40; 44; 45) zu erkennen, die für einen aggressiven Prostatatumor oder für einen nicht aggressiven Prostatatumor charakteristisch sind.

4. Vorrichtung nach einem der vorhergehenden Ansprüche,
wobei die elektronische Auswerteeinrichtung (20) eingerichtet ist, um eine örtliche Veränderung eines Raman-Signals (41-43), das für einen aggressiven Prostatatumor oder für einen nicht aggressiven Prostatatumor charakteristisch ist, aus den an der Mehrzahl von Positionen erfassten Raman-Spektren (36-39; 40; 44; 45) zu ermitteln.

5. Vorrichtung nach einem der vorhergehenden Ansprüche,
wobei die elektronische Auswerteeinrichtung (20) eingerichtet ist, um zum Bestimmen des Gradientenfeldes ortsaufgelöst einen Gradienten (50; 60) einer Intensität und/oder eines spektralen Gewichts des Raman-Signals zu ermitteln.

6. Vorrichtung nach einem der vorhergehenden Ansprüche ,
wobei die elektronische Auswerteeinrichtung (20) eingerichtet ist, um eine Position des Prostatatumors in der Gewebeprobe oder relativ zu der Gewebeprobe durch die Auswertung der an der Mehrzahl von Positionen erfassten Raman-Spektren (36-39; 40; 44; 45) zu ermitteln.

7. Vorrichtung nach einem der vorhergehenden Ansprüche,
wobei die elektronische Auswerteeinrichtung (20) eingerichtet ist, um die an einen Position oder der Mehrzahl von Positionen erfassten Raman-Spektren (36-39; 40; 44; 45) mit Informationen über gespeicherte Referenz-Spektren zu vergleichen.

8. Vorrichtung nach einem der vorhergehenden Ansprüche,
wobei die Vorrichtung (1) eingerichtet ist, um wenigstens einige der an der Mehrzahl von Positionen erfassten Raman-Spektren (36-39; 40; 44; 45) an unterschiedlichen subzellulären Strukturen von Prostatagewebezellen zu erfassen.

9. Vorrichtung nach einem der vorhergehenden Ansprüche,
wobei die Gewebeprobe (9) ein histologischer Schnitt von einer oder mehreren Prostatabiopsatstanzen ist.

10. Vorrichtung nach einem der vorhergehenden Ansprüche, wobei die Gewebeprobe (9) ein oder mehrere histologische Schnitte von einem Teil oder mehreren Teilen einer chirurgisch entfernten Prostata ist.

11. Vorrichtung nach einem der vorhergehenden Ansprüche,
wobei die Auswerteeinrichtung eingerichtet ist, um an der Gewebeprobe (9) erfasste Raman-Spektren mit Referenzspektren zu vergleichen, um eine Aggressivität oder Nichtaggressivität des Prostatatumors zu bestimmen.

12. Vorrichtung nach einem der vorhergehenden Ansprüche,
wobei die elektronische Auswerteeinrichtung (20) eingerichtet ist, um durch die Auswertung des wenigstens einen Raman-Spektrums (36-39; 40; 44; 45) eine Aggressivität des Prostatatumors zu bestimmen.

13. Verfahren zum Auswerten einer Gewebeprobe zum Erkennen eines Prostatatumors, umfassend
Erfassen wenigstens eines Raman-Spektrums (36-39; 40; 44; 45) der Probe und
Erkennen des Prostatatumors durch Auswerten des wenigstens einen Raman-Spektrums (36-39; 40; 44; 45),
wobei das Erfassen des wenigstens einen Raman-Spektrums umfasst:

Erfassen von Raman-Spektren (36-39; 40; 44; 45) an einer Mehrzahl von
Positionen der Gewebeprobe, und

wobei das Auswerten des wenigstens einen Raman-Spektrums umfasst:

Auswerten der an der Mehrzahl von Positionen erfassten Raman-Spektren (36-39; 40; 44; 45) um durch Ermittlung eines Gradientenfeldes aus den an der Mehrzahl von Positionen erfassten Raman-Spektren (36-39; 40; 44; 45) eine Position des Prostatatumors in der Gewebeprobe oder relativ zu der Gewebeprobe zu erkennen, wobei das Gradientenfeld für die an der Mehrzahl von Positionen (32-35) erfassten Raman-Spektren angibt, wie stark und in welcher Richtung sich eine Intensität eines Raman-Peaks oder mehrerer Raman-Peaks ändert wobei das Verfahren umfasst: Ausführen einer statistische Auswertung des wenigstens einen Raman-Spektrums (36-39; 40; 44; 45), um zu bestimmen, ob ein aggressiver oder nicht aggressiver Prostatatumor vorliegt.

14. Verfahren nach Anspruch 13,
wobei die statistische Auswertung eine Hauptkomponentenanalyse und/oder eine Clusteranalyse umfasst.

15. Verfahren nach einem der Ansprüche 13 bis 14,
wobei abhängig von den an der Mehrzahl von Positionen erfassten Raman-Spektren (36-39; 40; 44; 45) eine Verteilung wenigstens eines Biomarkers bestimmt wird, um

eine Position des Prostatatumors in der Gewebeprobe oder relativ zu der Gewebeprobe zu bestimmen, und/oder eine Aggressivität des Prostatatumors

zu bestimmen.

**Claims**

1. Device for identifying a prostate tumor, comprising
a Raman spectroscopy system (10) for acquiring at least one Raman spectrum (36-39; 40; 44; 45) of a tissue sample (9), and
an electronic evaluation unit (20) set up to identify the prostate tumor based on an evaluation of the at least one Raman spectrum (36-29; 40; 44; 45),
wherein the device is set up to acquiring at least one Raman spectrum (36-39; 40; 44; 45) at a plurality of positions (32-35) of the tissue sample (9), and
wherein the electronic evaluation unit (20) is set up to evaluate the Raman spectra (36-39; 40; 44; 45) acquired at the plurality of positions to determine a gradient field using the evaluation of the Raman spectra (36-39; 40; 44; 45) acquired at the plurality of positions (32-35) to determine a position of the prostate tumor in the tissue sample (9) or relative to the tissue sample (9), wherein the gradient field for the Raman spectra (36-39; 40; 44; 45), acquired at the plurality of positions, indicates how strongly and in which direction an intensity of a Raman peak or several Raman peaks changes,
wherein the electronic evaluation unit (20) is set up to determine whether an aggressive or non-aggressive prostate tumor is present using a statistical evaluation of the Raman spectra (36-39; 40; 44; 45) acquired at the plurality of positions.

2. Device according to claim 1, wherein the electronic evaluation unit (20) is set up to determine whether an aggressive or non-aggressive prostate tumor is present using a principal component analysis or a cluster analysis of the Raman spectra (36-39; 40; 44; 45) acquired at the plurality of positions.

3. Device according to any one of the preceding claims, wherein the electronic evaluation unit (20) is set up to identify Raman peaks and characteristic patterns in the Raman spectra (36-39; 40; 44; 45) acquired at the plurality of positions which are characteristic for an aggressive prostate tumor or for a non-aggressive prostate tumor by evaluating the Raman spectra (36-39; 40; 44; 45) acquired at a plurality of positions.

4. Device according to any one of the preceding claims, wherein the electronic evaluation unit (20) is set up to determine a local change of a Raman signal (41-43) which is characteristic for an aggressive prostate tumor or for a non-aggressive prostate tumor from the Raman spectra (36-39; 40; 44; 45) acquired at the plurality of positions.

5. Device according to any one of the preceding claims, wherein the electronic evaluation unit (20) is set up in order for determining, by spatial resolution, the gradient field to determine a gradient (50; 60) of an intensity and/or a spectra weight of the Raman signal.

6. Device according to any one of the preceding claims, wherein the electronic evaluation unit (20) is set up to determine a position of the prostate tumor in the tissue sample or relative to the tissue sample using the evaluation of the Raman spectra (36-39; 40; 44; 45) acquired at the plurality of positions.

7. Device according to any one of the preceding claims, wherein the electronic evaluation unit (20) is set up to compare the Raman Spectra (36-39; 40; 44; 45) acquired at one position or the plurality of positions with information of stored reference spectra.

8. Device according to any one of the preceding claims, wherein the device (1) is set up to acquire at least some of the Raman spectra (36-39; 40; 44; 45) acquired at the plurality of positions on different sub-cellular structures of prostatic tissue cells.

9. Device according to any one of the preceding claims, wherein the tissue sample (9) is a histological section of one or more prostate biopsy punch/punches.

10. Device according to any one of the preceding claims, wherein the tissue sample (9) is one or more histological section/sections of one or more portion/portions of a surgically removed prostate.

11. Device according to any one of the preceding claims, wherein the evaluation unit is set up to compare a Raman spectra acquired from the tissue sample (9) with reference spectra to determine aggressiveness or non-aggressiveness of the prostate tumor.

12. Device according to any one of the preceding claims, wherein the electronic evaluation unit (20) is set up to determine aggressiveness of the prostate tumor by evaluating the at least one Raman spectrum (36-39; 40; 44; 45).

13. Method for evaluating of a tissue sample for determining a prostate tumor, comprising
acquiring at least one Raman spectrum (36-39; 40; 44; 45) of the sample and
identifying the prostate tumor by evaluating the at least one Raman spectrum (36-39; 40; 44; 45),
wherein the acquiring of at least one Raman spectrum comprises:
acquiring of Raman spectra (36-39; 40; 44; 45) at a plurality of positions of the tissue sample, and
wherein the evaluation of the at least one Raman spectrum comprises:

evaluating the Raman spectra acquired at the plurality of positions (36-39; 40; 44; 45) to identify a position of the prostatic tumor in the tissue sample or relative to the tissue sample by determining a gradient field from the Raman spectra (36-39; 40; 44; 45) acquired at the plurality of positions, wherein the gradient field for the Raman spectra (36-39; 40; 44; 45) acquired at the plurality of positions (32-35) indicates how strongly and in which direction an intensity of a Raman peak or several Raman peaks changes,
wherein the method comprises: performing a statistic evaluation of the at least one Raman spectrum (36-39; 40; 44; 45) to determine if an aggressive or non-aggressive prostate tumor is present.

14. Method according to claim 13,
wherein the statistical evaluation comprises a principal component analysis and/or a cluster analysis.

15. Method according to any one of claims 13 to 14,
wherein distribution of at least one biomarker is determined, depending on the Raman spectra (36-39; 40; 44; 45) acquired at the plurality of positions, to determine a position of the prostate tumor in the tissue sample or relative to the tissue sample, and/or to determine the aggressiveness of the prostate tumor.

**Revendications**

1. Dispositif de diagnostic d'une tumeur de la prostate, comprenant un système de spectroscopie Raman (10) pour détecter au moins un spectre Raman (36-39 ; 40 ; 44 ; 45) d'un échantillon de tissu (9), et
un appareil d'analyse électronique (20) qui est configuré pour diagnostiquer la tumeur de la prostate en fonction d'une analyse du au moins un spectre Raman (36-39 ; 40 ; 44 ; 45),
dans lequel le dispositif est conçu pour détecter au moins un spectre Raman (36-39 ; 40 ; 44 ; 45) dans une pluralité de positions (32-35) de l'échantillon de tissu (9), respectivement
dans lequel l'appareil d'analyse électronique (20) est configuré pour analyser les spectres Raman (36-39 ; 40 ; 44 ; 45) détectés dans la pluralité de positions afin de déterminer un champ de gradient par l'analyse des spectres Raman (36-39 ; 40 ; 44 ; 45) détectés dans la pluralité de positions pour identifier une position de la tumeur de la prostate dans l'échantillon de tissu (9) ou par rapport à l'échantillon de tissu (9), dans lequel le champ de gradient pour les spectres Raman détectés dans la pluralité de positions (32-35) indique dans quelle mesure et dans quelle direction une intensité d'un pic Raman ou de plusieurs pics Raman change,

dans lequel l'appareil d'analyse électronique (20) est conçu pour déterminer si une tumeur de la prostate agressive ou non agressive est présente par une analyse statistique des spectres Raman (36-39 ; 40 ; 44 ; 45) détectés dans la pluralité de positions.

2. Dispositif selon la revendication 1,
dans lequel l'appareil d'analyse électronique (20) est conçu pour déterminer si une tumeur de la prostate agressive ou non agressive est présente par une analyse en composantes principales ou une analyse en groupements des spectre Raman (36-39 ; 40 ; 44 ; 45) détectés dans la pluralité de positions.

3. Dispositif selon l'une quelconque des revendications précédentes,
dans lequel l'appareil d'analyse électronique (20) est conçu pour, par l'analyse des spectres Raman (36-39 ; 40 ; 44 ; 45) détectés dans la pluralité de positions (, diagnostiquer dans les spectres Raman (36-39 ; 40 ; 44 ; 45) détectés dans la pluralité de positions des pics Raman et des motifs caractéristiques qui sont caractéristiques d'une tumeur de la prostate agressive ou d'une tumeur de la prostate non agressive.

4. Dispositif selon l'une quelconque des revendications précédentes,
dans lequel l'appareil d'analyse électronique (20) est conçu pour détecter une variation locale d'un signal Raman (41-43), qui est caractéristique d'une tumeur de la prostate agressive ou d'une tumeur de la prostate non agressive, à partir des spectres Raman (36-39, 40, 44, 45) détectés dans la pluralité de positions.

5. Dispositif selon l'une quelconque des revendications précédentes,
dans lequel l'appareil d'analyse électronique (20) est conçu pour, dans le but de déterminer le champ de gradient, déterminer avec une résolution spatiale un gradient (50 ; 60) d'une intensité et/ou d'un poids spectral du signal Raman .

6. Dispositif selon l'une quelconque des revendications précédentes,
dans lequel l'appareil d'analyse électronique (20) est conçu pour déterminer une position de la tumeur de la prostate dans l'échantillon de tissu ou par rapport à l'échantillon de tissu via l'analyse des spectres Raman (36-39 ; 40 ; 44 ; 45) détectés dans la pluralité de positions.

7. Dispositif selon l'une des revendications précédentes,
dans lequel l'appareil d'analyse électronique (20) est conçu pour comparer les spectres Raman (36-39 ; 40 ; 44 ; 45) détectés dans une ou dans la pluralité de positions avec des informations concernant des spectres de référence stockés.

8. Dispositif selon l'une quelconque des revendications précédentes,
dans lequel le dispositif (1) est conçu pour détecter au moins certains des spectres Raman (36-39 ; 40 ; 44 ; 45) détectés dans la pluralité de positions au niveau de différentes structures subcellulaires de cellules du tissu prostatique.

9. Dispositif selon l'une quelconque des revendications précédentes,
dans lequel l'échantillon de tissu (9) est une coupe histologique d'un ou plusieurs poinçons de biopsie de la prostate.

10. Dispositif selon l'une quelconque des revendications précédentes,
dans lequel l'échantillon de tissu (9) est une ou plusieurs coupes histologiques d'une ou de plusieurs parties d'une prostate prélevée chirurgicalement.

11. Dispositif selon l'une quelconque des revendications précédentes,
dans lequel l'appareil d'analyse est conçu pour comparer les spectres Raman détectés au niveau de l'échantillon de tissu (9) avec des spectres de référence afin de déterminer l'agressivité ou la non-agressivité de la tumeur de la prostate.

12. Dispositif selon l'une quelconque des revendications précédentes,
dans lequel l'appareil d'analyse électronique (20) est conçu pour déterminer l'agressivité de la tumeur de la prostate par l'analyse d'au moins un spectre Raman (36-39 ; 40 ; 44 ; 45).

**13.** Procédé d'analyse d'un échantillon de tissu pour diagnostiquer une tumeur de la prostate, comprenant les étapes consistant à

détecter au moins un spectre Raman (36-39 ; 40 ; 44 ; 45) de l'échantillon, et

diagnostiquer la tumeur de la prostate en évaluant au moins un spectre Raman (36-39 ; 40 ; 44 ; 45),

dans lequel la détection du au moins un spectre Raman comprend les étapes consistant à :

détecter des spectres Raman (36-39 ; 40 ; 44 ; 45) dans une pluralité de positions de l'échantillon de tissu, et

dans lequel l'analyse du au moins un spectre Raman comprend les étapes consistant à :

analyser les spectres Raman (36-39 ; 40 ; 44 ; 45) détectés dans la pluralité de positions pour diagnostiquer, par une identification d'un champ de gradient à partir des spectres Raman (36-39 ; 40 ; 44 ; 45) détectés dans la pluralité de positions, une position de la tumeur de la prostate dans l'échantillon de tissu ou par rapport à l'échantillon de tissu, dans lequel le champ de gradient pour les spectres Raman détectés dans la pluralité de positions (32-35) indique dans quelle mesure et dans quelle direction une intensité d'un pic Raman ou de plusieurs pics Raman change,

le procédé comprenant l'étape consistant à : effectuer une analyse statistique du au moins un spectre Raman (36-39 ; 40 ; 44 ; 45) afin de déterminer si une tumeur de la prostate agressive ou non agressive est présente.

**14.** Procédé selon la revendication 13,

dans lequel l'analyse statistique comprend une analyse en composantes principales et/ou une analyse en groupements.

**15.** Procédé selon l'une quelconque des revendications 13 à 14,

dans lequel une distribution d'au moins un biomarqueur est déterminée en fonction des spectres Raman (36-39 ; 40 ; 44 ; 45) détectés dans la pluralité de positions pour

déterminer une position de la tumeur de la prostate dans l'échantillon de tissu ou par rapport à l'échantillon de tissu, et/ou

déterminer l'agressivité de la tumeur de la prostate.

FIG. 1

30

32    33

34    35

31

FIG. 2

36    37    38    39

31

FIG. 3

FIG. 4

FIG. 5

FIG. 6

FIG. 7

FIG. 8

FIG. 9

FIG. 10

FIG. 11

FIG. 12

FIG. 13

Intensität der Raman-Peak

82

83

x

FIG. 14

90

| Erfassen von Raman-Spektren | 91 |

Identifizieren von Biomarkern zugeordneten Raman-Peaks — 92

Bestimmung einer örtlicher Veränderung des Gewichts der Raman-Peaks — 93

Ermitteln der Aggressivität des Prostatatumors — 94

FIG. 15